# EUROPEAN PATENT SPECIFICATION

(11) **EP 2 959 665 B1**
(45) Date of publication and mention of the grant of the patent: **15.07.2020**
(21) Application number: 14753547.0
(22) Date of filing: 24.02.2014
(51) Int. Cl.: H04L 29/12, A61N 1/00

(54) **APPARATUSES FOR NETWORKING NEUROMODULATION OF A GROUP OF INDIVIDUALS**
VORRICHTUNGEN FÜR NEUROMODULATIONSVERNETZUNG EINER GRUPPE VON PERSONEN
APPAREILS PERMETTANT LA MISE EN RÉSEAU DE LA NEUROMODULATION D'UN GROUPE D'INDIVIDUS

(30) Priority: 22.02.2013 US 201361767945 P; 28.02.2013 US 201361770479 P; 10.09.2013 US 201361875981 P; 06.11.2013 US 201361900880 P
(43) Date of publication of application: 30.12.2015
(73) Proprietor: Cerevast Medical, Inc., Bothell, WA 98011 (US)
(72) Inventor: WETMORE, Daniel, Z., Los Gatos, CA 95030 (US); GOLDWASSER, Isy, Los Gatos, CA 95030 (US); CHARLESWORTH, Jonathan, Los Gatos, CA 95030 (US); PAL, Sumon, K., Los Gatos, CA 95030 (US); TYLER, William, J., Los Gatos, CA 95030 (US); SATO, Tomokazu, Los Gatos, CA 95030 (US)
(74) Representative: Lohr, Jöstingmeier & Partner
(86) International application number: PCT/US2014/018061
(87) International publication number: WO 2014/130960

(56) References cited:
- US-A1- 2007 123 952
- US-A1- 2009 210 028
- US-A1- 2010 042 180
- US-A1- 2011 288 610
- US-A1- 2011 288 610
- US-A1- 2012 029 591
- US-A1- 2012 182 924
- US-A1- 2012 245 653
- US-A1- 2012 289 869
- US-A1- 2012 296 390

## Description

### FIELD

The present invention relates to the coordination of transcranial neuromodulation for a group of individuals; the transcranial neuromodulation may be via electrical stimulation (TES), transcranial ultrasound neuromodulation, another form of neuromodulation, or a combination of multiple forms of neuromodulation. In particular, described herein are apparatuses for safely and securely networking transcranial neuromodulation devices each worn by an individual in a group so that the individuals may share a similar cognitive effect from the coordinated transcranial neuromodulation.

### BACKGROUND

The brain is composed of neurons and other cell types in connected networks that process sensory input, generate motor commands, and control all other behavioral and cognitive functions. Noninvasive neuromodulation technologies that modulate neural activity can induce altered behavior, cognitive states, perception, and motor output. Although systems and methods for noninvasive neuromodulation of an individual have been proposed, the coordinated neuromodulation of a group of individuals has not previously been developed. Existing apparatuses, including transcranial electric stimulation (TES) or ultrasound neuromodulation apparatuses lack the capability to safely coordinate neuromodulation of a group of individuals. Coordinated neuromodulation of a group (e.g. more than two individuals, more than three individuals, etc.) may provide numerous benefits, including therapeutic effects, enhancing of amusement/entertainment experiences, and enhancing performance in a group observational or participation event. For example, coordinated neuromodulation may enhance the experience for an audience at a musical performance or dramatic performance by inducing and/or enhancing a cognitive state relevant to the perception of the event in a positive and beneficial manner.

Existing TES apparatuses generally operate through scalp electrodes to affect brain function using transcranial alternating current stimulation (tACS), transcranial direct current stimulation (tDCS), and transcranial random noise stimulation (tRNS). Relative to tDCS, tACS and tRNS offer the advantage of reductions in pain, tingling, and other side effects on the scalp. Another strategy to reduce side effects is to use a high-density-tDCS (HD-tDCS) system with smaller electrode pads, such as ones sold by Soterix Medical. tACS also has the advantage of being inherently temporal in nature and thus capable of affecting, inducing, or destructively interfering with endogenous brain rhythms.

TES has been shown to be advantageous for modulating brain activity and cognitive function in individuals, such as improving motor control and motor learning, improving memory consolidation during slow-wave sleep, regulating decision-making and risk assessment, affecting sensory perception, and causing movements. Systems and methods for TES have been disclosed (see for example, patents: US 4,646,744; US 5,540,736; US 8,190,248; US 8,239,030; and US patent applications US 2011/0144716 and US 2009/0177243). Other systems described in the prior art require surgical implantation of components for electrical stimulation on the head of a user (see for example patents US 8,121,695 and US 8,150,537). tDCS systems with numerous electrodes and a high level of configurability have been disclosed (see, for example, patent application: US 2012/0209346, US 2012/0265261, and US 2012/0245653), as have portable TES systems for auto-stimulation (US 8,554,324).

Hardware and software systems for TES may include: a battery or power supply safely isolated from main power; a current regulator to supply constant current as the impedance between an electrode and a subject's head changes slightly (e.g. due to movement, sweating, etc.); and circuitry to ensure that spikes of current do not pass into the subject; control hardware and/or software for triggering a TES event and controlling the waveform, duration, intensity, and other parameters of stimulation of each electrode; and one or more pairs of electrodes with gel, saline, or another material for electrical coupling to the scalp.

The simplest form of TES is tDCS. tACS requires additional hardware to deliver alternating currents to the electrodes at an appropriate frequency. tRNS additionally requires a microcontroller or other processor configured to provide random values with appropriate structure that are then converted to an analog signal and used to gate current at a the desired intensity (e.g. at a desired amplitude, frequency, and/or duration) through appropriate circuitry. Electrical stimulation waveforms of arbitrary complexity can be generated by a controllable current source of a TES system and used to induce beneficial cognitive effects.

Another form of non-invasive transcranial neuromodulation includes ultrasound neuromodulation. Ultrasound (US) has been used for many medical applications, and is generally known as cyclic sound pressure with a frequency greater than the upper limit of human hearing. An important benefit of ultrasound therapy is its non-invasive nature. US waveforms can be defined by their acoustic frequency, intensity, waveform duration, and other parameters that vary the time course of acoustic waves in a target tissue. Transcranial ultrasound neuromodulation has been shown to activate, inhibit, or modulate neuronal activity (e.g., US 8,591,419 and patent applications US 20070299370, US 20110092800, US 2008/0045882, US 2011/0178441, and WO/2011/057028). To affect brain function, transcranial ultrasound neuromodulation typically requires appropriate ultrasound waveform parameters, including acoustic frequencies generally less than about 10 MHz, spatial-peak temporal-average intensity generally less than about 1 W/cm², and appropriate pulsing and other waveform characteristics to ensure that heating of a targeted brain region does not exceed about 2 degrees Celsius for more than about 5 seconds. Transcranial ultrasound neuromodulation induces neuromodulation primarily through vibrational or mechanical mechanisms. Noninvasive and nondestructive transcranial ultrasound neuromodulation is in contrast to other transcranial ultrasound based techniques that use a combination of parameters to disrupt, damage, destroy, or otherwise affect neuronal cell populations so that they do not function properly and/or cause heating to damage or ablate tissue.

While prior systems have been developed to allow for TES and transcranial ultrasound neuromodulation, the systems currently available typically have been designed for application and usage in a specific setting, such as a medical or clinical setting, with application, monitoring and use by trained staff and/or medical professionals. Nothing in these systems allow for remote control and operation of a transcranial neuromodulation system. Further, the coordinated neuromodulation of a group of individuals has not been addressed. The coordinated neuromodulation of a group of individuals offers additional challenges not contemplated by traditional single-patient (one individual) neuromodulation within a clinical setting.

In general, there are numerous situations and applications outside of the clinical setting where noninvasive transcranial neuromodulation would be advantageous, particularly when applied in a coordinated manner to a group of individuals. However, the systems currently available are typically limited in application and design in ways that prevent regular (e.g., periodic) non-clinical /portable use. For example, current apparatuses typically are not self-contained, cannot be controlled remotely, may be difficult for non-medical professionals to apply and generally are not easily portable. Most significantly, such devices cannot be networked, and in particular, cannot be networked securely in a manner that safely and effectively allows coordinated neuromodulation of a group of individuals, for example, synchronizing or otherwise monitoring and controlling neuromodulation between two or more individuals in a social setting.

The apparatuses (including systems, devices and assemblies) for achieving noninvasive transcranial neuromodulation described herein may address these limitations and allow for the regular (including daily, weekly, monthly) or occasional use portable neuromodulation apparatuses, such as TES and/or transcranial ultrasound neuromodulation devices. The apparatuses described herein may address the problems with the effectiveness, simplicity, security, privacy, and triggering of transcranial neuromodulation in both individual-use operation and the coordinated neuromodulation of a group of individuals.

US 2011/288610 A1 discloses a mobile device for transcranial auto-stimulation and a method for controlling and regulating the device. The disclosure relates to a mobile device for transcranial auto-stimulation, controlled according to need, of circumscribed brain structures and brain systems, and to a method for controlling and regulating the device The device relates in particular to a device for transcranial electric current stimulation, comprising the following components: electrodes with fastening means for exactly positioning on the skin of the head and electrical connecting lines and a transportable, miniaturized stimulation generator comprising a current generator, a controller, a user interface, an electrical energy storage device and a monitoring and safety module with a separate electrical energy storage device.

### SUMMARY OF THE DISCLOSURE

According to a first aspect of the present invention there is provided the networkable transcranial neuromodulation system of claim 1. Additional aspects of the invention are set out in the dependent claims. Aspects, embodiments, examples, and methods of the present disclosure that do not fall within the scope of the appended claims do not form part of the invention and are merely provided for illustrative purposes.

Described herein are apparatuses and methods for noninvasive (e.g., transcranial, transdermal) neuromodulation that may be used to coordinate neuromodulation of an entire group of individuals. For example, described herein are apparatuses that may be securely networked so that they can be wirelessly and remotely controlled in a secure and safe manner to coordinate neuromodulation among group members. In general an apparatus may include a device, system, or any other assembly. These apparatuses may be configured to operate autonomously, e.g., without requiring coordination or outside control, or coordinated by outside control, or semi-autonomously, e.g., with minimal or initial outside control; the apparatuses may be configured to switch between autonomous and controlled (and/or semi-autonomous) operation in a secure manner. Any of these apparatuses may be configured as a wearable, self-contained, self-powered, and/or self-coupling, ("puck") apparatus that is configured for wireless communication. A system for coordinated neuromodulation may include one or more of such transcranial neuromodulation apparatuses. A system for coordinated neuromodulation may also or alternatively include a third-party controller that coordinates the application of neuromodulation by multiple neuromodulation applicators being worn by different individuals. In general, a third-party controller may be separate from one or more of the wearable applicators, or it may be associated with one of the wearable applicators (pucks) that it controls; the third-party controller may be hardware, software, firmware, or some combination thereof. For example, a third-party controller may be an application ("app") that configures a microprocessor (such as a desktop, laptop, pad, smartphone, etc.) to coordinate neuromodulation of one or a group of neuromodulation apparatuses.

In general any of the neuromodulation apparatuses described herein may be configured to modulate neuronal activity by a noninvasive mechanism, including but not limited to transcranial electrical stimulation (TES) and transcranial ultrasound neuromodulation, or a combination of the two.

As mentioned, a transcranial neuromodulation apparatus as described herein may be self-adhesive to the head, self-powered (by one or more power source, e.g., battery), and self-contained, containing all components in a single housing, and may be referred to as a 'puck'. A transcranial ultrasound neuromodulation puck may also be self-coupling, and may be disposable or semi-disposable. A transcranial neuromodulation apparatus as described herein generally includes components for wireless communication including a receiver, transmitter, transceiver, etc.

Any of the apparatuses described herein may be securely networked. In general, these apparatuses may be configured to wirelessly communicate with a third-party controller and/or other neuromodulation apparatuses. Safe operation of the apparatuses may be assured in some instances by providing addressability (e.g., unique static or dynamic identifiers specific to particular apparatuses in a group), encryption of command information and/or data specific to an individual user, and/or security keying/provisioning the devices and any third-party controller. For example, a neuromodulation apparatus may be addressable. Addressability may identify a particular instance of a neuromodulation system from other instances so that a particular neuromodulation system can be located, identified, and communicated with in a targeted manner. Thus, a neuromodulation system may generally operate in a secure and private manner. Security may be an important neuromodulation system feature to ensure that the apparatus is not remotely triggered accidentally or contrary to the wishes of a user, and to maintain all data stored locally on the neuromodulation apparatus and transmitted to and from the neuromodulation apparatus privately according to the wishes of the user.

In general, the coordinated neuromodulation of a group of individuals may be accomplished using networked/networkable neuromodulation apparatuses. A neuromodulation apparatus may be configured for synchronization with other neuromodulation apparatuses that are wearably attached to other users. Coordinated neuromodulation to achieve paired, group, and social applications of neuromodulation may be referred to as "social neuromodulation". Coordinated neuromodulation may include simultaneous/synchronized neuromodulation, e.g., neuromodulation that is timed to apply similar or identical neuromodulation to a plurality of individuals wearing neuromodulation apparatuses. Coordinated neuromodulation includes applying identical neuromodulation signals or waveforms (e.g., TES and/or ultrasound signals/waveforms), or applying neuromodulation signals/waveforms that achieve the same or similar effects in each of the participating individuals. A third-party controller may transmit the actual waveforms, or it may transmit instructions to apply such waveforms (or equivalent/similar waveforms) stored locally on the neuromodulation apparatus.

Although many of the examples described herein assume that each member of a group of individuals is each wearing a separate neuromodulation apparatus, in some variations a neuromodulation system may be shared between two or more individuals.

Any of the neuromodulation apparatuses described herein may be configured/adapted to include scheduling of neuromodulation. For example, a neuromodulation apparatus may be configured for scheduling a neuromodulation session. The application of neuromodulation may be triggered locally or remotely.

Any of the neuromodulation apparatuses described may include one or more sensors. Thus, a neuromodulation system may incorporate or receive data from one or more sensors that determine an individual's location or proximity to another individual wearing a neuromodulation apparatus. Alternatively or additionally, a neuromodulation system may incorporate or receive data from one or more sensors for recording brain activity. A neuromodulation system may incorporate or receive data from one or more sensors for measuring physiology (e.g., heart rate, galvanic skin response, temperature, etc.).

Numerous cognitive effects of TES have been described, and TES is an active field of scientific research. The TES apparatuses described may achieve neuromodulation to affect learning and memory, attention, creativity, decision-making, and other cognitive states. In general the apparatuses described herein are neuromodulation apparatuses (e.g., TES, ultrasound, etc.) that may non-invasively and transcranially apply energy to modulate neuronal activity, e.g. by stimulation and/or inhibition. Thus, neuromodulation may in some contexts be referred to as neurostimulation. For example, any of the neuromodulation apparatuses described may be configured as a TES applicator that induce neuromodulation, including transcranial direct current stimulation (tDCS), transcranial alternating current stimulation (tACS), cranial electrotherapy stimulation (CES), transcranial random noise stimulation (tRNS), and other electrical stimulation waveforms of arbitrary complexity.

Any of the neuromodulation systems described herein may include two types of assemblies that function together to control and deliver transcranial neuromodulation protocols to a user. A first assembly, which may be referred to as a "neuromodulation controller assembly" or "controller assembly", may be at least partially housed within a housing and may incorporate electronic circuitry for driving transcranial neuromodulation, a battery to supply power, a wireless transmitter and receiver module (e.g., transceiver), and other optional features. The controller assembly may be wearably attached to a user on the head, face, neck, or another portion of the body near the head. A second assembly, which may be referred to as a "neuromodulation delivery assembly" or "delivery assembly", may self-adhere to the head, and connects to the controller assembly (e.g., by a cable or wire) to receive energy, transmit control signals to the delivery assembly, and transmit other data from the delivery assembly. Delivery assemblies may be configured to be disposable and/or interchangeable. Neuromodulation apparatuses as described herein may comprise a single controller assembly and one or more delivery assemblies.

For example, a neuromodulation apparatus configured to deliver TES may include a delivery assembly that incorporates one or more electrodes for delivering current to the scalp to achieve TES. A delivery assembly may incorporate one or more ultrasound transducers acoustically coupled to the user's head. A delivery assembly may incorporate both TES electrodes and one or more ultrasound transducers. A deliver assembly may be self-adherent and/or self-coupling.

A controller assembly, delivery assembly, and an electrical interface connecting the controller assembly to a delivery assembly may also include optional features that improve the wearability, comfort, interchangeability, and flexible brain region targeting of any of the transcranial neuromodulation systems described herein.

For example, described herein are networkable transcranial neuromodulation systems adapted to apply coordinated neuromodulation to a group of individuals. A networkable transcranial neuromodulation system may include: a first transcranial neuromodulator apparatus comprising: a first wireless receiver and transmitter module connected to a first controller that is configured to apply transcranial neuromodulation from a first applicator; a second transcranial neuromodulator apparatus: a second wireless receiver and transmitter module connected to a second controller that is configured to apply transcranial neuromodulation from a second applicator; and a third-party controller configured to transmit control information to the first and second transcranial neuromodulator apparatuses to coordinate neuromodulation by the first and the second transcranial neuromodulator apparatus.

A networkable transcranial neuromodulation system may also alternatively or additionally include: a first transcranial neuromodulator apparatus configured to adhesively secure to a first individual's head, the first transcranial neuromodulator apparatus comprising: a first power source, a first wireless receiver and transmitter module, a first applicator configured to deliver transcranial neuromodulation and a first controller configured to receive instructions from the first wireless receiver and transmitter module and to apply transcranial neuromodulation from the first applicator; a second transcranial neuromodulator apparatus configured to adhesively secured to a second individual's head, the second transcranial neuromodulator apparatus comprising: a second power source, a second wireless receiver and transmitter module, a second applicator configured to deliver transcranial neuromodulation and a second controller configured to receive instructions from the second wireless receiver and transmitter module and to apply transcranial neuromodulation from the second applicator; and a third-party controller configured to transmit control information to the first and second transcranial neuromodulator apparatuses to coordinate neuromodulation by the first and the second transcranial neuromodulator apparatus.

As mentioned, any of the apparatuses described herein may be adhesive, or partially adhesive. For example, the entire apparatus may be adhesively secured to a subject (e.g., to the subject's head, neck, etc.). In some variations, the apparatus is partially adhesive, so that a portion of the apparatus (such as one or more applicators) is adhesively secured to the subject while another portion (e.g., a controller assembly) is non-adherently attached to the subject, for example by clipping onto a lapel or clothing and connecting (e.g., by wires) to the applicator(s) such as electrodes and/or ultrasound couplant.

In any of these systems, more than the first and second (e.g., third, fourth, fifth, etc.) transcranial neuromodulation apparatuses may be included. The transcranial neuromodulation apparatuses may be configured as transcranial ultrasound neuromodulation apparatuses (e.g., wherein the first and second applicators comprise ultrasound transducers), transcranial electrical stimulation (TES) apparatuses (e.g., wherein the first and second applicators each comprise two or more electrodes), and/or both. Each transcranial neuromodulation apparatus may comprise a unique address and the third party controller may be configured to transmit control information using the unique addresses.

In general, any of the transcranial neuromodulation apparatuses may include a security module configured to prevent the control of neuromodulation by the third-party controller unless the security module has received a valid security key from the third-party controller. For example, the first transcranial neuromodulation apparatus comprises a first security module configured to prevent the control of neuromodulation by the third-party controller unless the first security module has received a valid security key from the third-party controller, and the second transcranial neuromodulation apparatus may comprise a second security module configured to prevent the control of neuromodulation by the third-party controller unless the second security module has received a valid security key from the third-party controller.

For example, also described herein are networkable transcranial neuromodulation apparatuses adapted to securely apply coordinated neuromodulation to one individual of a group of individuals receiving coordinated neuromodulation, the apparatus comprising: an applicator configured to deliver transcranial neuromodulation; a wireless receiver and transmitter module; a controller configured to control the application of transcranial neuromodulation by the applicator; and a security module configured to determine if a valid security key has been received by the wireless receiver and transmitter module; wherein the controller is further configured to receive instructions from the wireless receiver and transmitter module and to apply transcranial neuromodulation based on the received instructions when the security module has determined that a valid security key was received.

Also described herein are networkable transcranial neuromodulation apparatuses adapted to apply coordinated neuromodulation to one individual of a group of individuals receiving coordinated neuromodulation, the apparatus comprising: a housing at least partially enclosing: a power source, a controller, and a wireless receiver and transmitter module; an applicator surface comprising: an adhesive configured to secure the device to the individual's head, and an applicator coupled to the controller and configured to deliver transcranial neuromodulation; and a security module configured to determine if a valid security key has been received by the wireless receiver and transmitter module; wherein the controller is configured to receive instructions from the wireless receiver and transmitter module and apply transcranial neuromodulation from the applicator based on the received instructions when the security module has determined that a valid security key was received.

The controllers of any or all of the transcranial neuromodulation apparatuses may each be configured to operate autonomously when not receiving control information from the third-party controller. For example, each transcranial neuromodulation apparatus may operate in an un-networked capacity, as an autonomous neuromodulator that can be controlled directly by the individual wearing the apparatus, including by using an application (e.g., on a wirelessly connected device such as the wearer's smartphone) to control the neuromodulator.

In general, the third-party controller may be configured to receive status information from each of the first and second transcranial neuromodulator apparatuses. Status information may indicate that the individual is ready to receive neuromodulation and/or may include any additional information about the status of the neuromodulator and/or the individual wearing the neuromodulator such as the quality of the contact between the apparatus and the individual (e.g., electrical and/or acoustic impedance, etc.). A third-party controller may be configured to determine if a transcranial neuromodulator apparatus is within range of wireless communication.

In general, a third-party controller may be configured to coordinate neuromodulation of each individual of a group (e.g., from the first and second transcranial neuromodulator apparatuses) by sending control instructions, which may be encrypted, and/or may include specific authentication information/security keys. For example, coordination may include instructing the transcranial neuromodulator apparatuses (e.g., the first and second transcranial neuromodulator apparatuses) to concurrently apply transcranial neuromodulation, and/or to all apply the same transcranial neuromodulation signal, or to apply a characteristic type of neuromodulation to each member of the group, either synchronously or non-synchronously (including with a delay for some participants, etc.). For example, the third-party controller may instruct the first and second transcranial neuromodulator apparatuses to concurrently apply the same transcranial neuromodulation signal to all of the transcranial neuromodulator apparatuses at the same time.

The third party control may be a device and/or may include a non-transitory computer-readable storage medium storing a set of instructions capable of being executed by a control processor, that when executed by the control processor causes the control processor to wirelessly communicate with a plurality of transcranial neuromodulator apparatuses. For example, the third-party control may include a non-transitory computer-readable storage medium storing a set of instructions capable of being executed by a smartphone, laptop, tablet, etc.

Also described herein are networkable transcranial neuromodulation apparatuses adapted to apply coordinated neuromodulation to one individual of a group of individuals each receiving coordinated neuromodulation. For example, a networkable transcranial neuromodulation apparatus adapted to apply coordinated neuromodulation may include: an applicator configured to deliver transcranial neuromodulation; a wireless receiver and transmitter module; and a controller configured to switch between an autonomous operation mode and a coordinated operation mode; wherein in the coordinated operation mode the controller is configured to receive instructions from a third-party controller through the wireless receiver and transmitter module and to apply transcranial neuromodulation based on the received instructions.

Alternatively or additionally, a networkable transcranial neuromodulation apparatus adapted to apply coordinated neuromodulation may include: a housing at least partially enclosing: a power source, a wireless receiver and transmitter module, and a controller configured to switch between an autonomous operation mode and a coordinated operation mode; and an applicator surface comprising: an adhesive configured to secure the device to the individual's head, and an applicator configured to deliver transcranial neuromodulation; wherein in the coordinated operation mode the controller is configured to receive instructions from a third-party controller through the wireless receiver and transmitter module and to apply transcranial neuromodulation based on the received instructions.

In any of the transcranial neuromodulation apparatuses described, the controller may also include a security module configured to determine if a valid security key has been received by the wireless receiver and transmitter module and to permit switching to the coordinated operation mode only when the valid security key has been received.

Any of the transcranial neuromodulation apparatuses described may also include a unique address associated with the apparatus, wherein the controller is configured to apply transcranial neuromodulation based on the received instructions during the coordinated operation mode only when the received instructions specify the unique address associated with the apparatus.

As mentioned above, any of the transcranial neuromodulation apparatuses described may be configured as transcranial ultrasound neuromodulation apparatuses, transcranial electrical stimulation (TES) apparatuses, as combined transcranial electrical stimulation and transcranial ultrasound apparatuses, or as a system that uses another form of neuromodulation. These apparatuses may transmit a ready status indicator when the apparatus is ready to receive instructions from the third-party controller.

Any of the transcranial neuromodulation apparatuses described may include a sensor configured to detect a physiological parameter from the individual (e.g., detecting brain activity, heart rate, etc.), and/or a sensor for detecting the connection between the apparatus and the individual (e.g., confirming that the apparatus is attached, and/or the quality of the contact, e.g., by impedance).

Also described herein are methods of coordinating neuromodulation of a plurality of individuals. For example, a method of coordinating neuromodulation of a plurality of individuals may include: receiving a first status indicator from a first transcranial neuromodulation apparatus being worn by a first individual; receiving a second status indicator from a second transcranial neuromodulation apparatus being worn by a second individual; and applying coordinated transcranial neuromodulation to both the first individual and the second individual.

A method of coordinating neuromodulation of a plurality of individuals may also include: receiving a first status indicator that is wirelessly transmitted from a first transcranial neuromodulation apparatus being worn by a first individual, wherein the first transcranial neuromodulation apparatus is a self-contained, self-powered and self-adherent apparatus; receiving a second status indicator that is wirelessly transmitted from a second transcranial neuromodulation apparatus being worn by a second individual, wherein the second transcranial neuromodulation apparatus is a self-contained, self-powered and self-adherent apparatus; and applying coordinated transcranial neuromodulation to both the first individual and the second individual.

In general, applying coordinated transcranial neuromodulation may include wirelessly transmitting control information controlling neuromodulation by both the first transcranial neuromodulation apparatus and the second transcranial neuromodulation apparatus. The control information may include start/stop times, durations of neuromodulation on/off periods, types of neuromodulation to apply (e.g., neuromodulation to increase relaxation, enhance attention, enhance energy, etc.), including in some variations neuromodulation waveforms, and the like.

Applying coordinated transcranial neuromodulation may include applying coordinated transcranial electrical stimulation (TES) and/or coordinated transcranial ultrasound stimulation; in some variations, the third-party controller is ambivalent between TES and ultrasound (or another form of neuromodulation), instructing just the type of neuromodulation, and relying on the local transcranial neuromodulation apparatus to determine the modality (and likely the waveforms) appropriate to that apparatus.

Applying coordinated transcranial neuromodulation may include applying the neuromodulation after all status indicators indicate that the group members are ready (e.g., after the first status indicator indicates that the first individual is ready to receive neuromodulation and after the second status indicator indicates that the second individual is ready to receive neuromodulation). In some variations the method (and corresponding apparatuses) may be configured so that individuals may "join" an ongoing coordinated neuromodulation already in progress on one or more neuromodulation apparatuses being worn by individuals in a group.

Applying coordinated transcranial neuromodulation may include concurrently (e.g., simultaneously) applying the neuromodulation to all members of the group (e.g., both the first individual and the second individual, etc.). As mentioned above, applying coordinated transcranial neuromodulation may include applying the same neuromodulation to both the first individual and the second individual.

Any of the methods described herein may include establishing a secure connection with each of the transcranial neuromodulation apparatuses in the group (e.g., both a first transcranial neuromodulation apparatus and a second transcranial neuromodulation apparatus where there are two apparatuses forming the group). Applying coordinated transcranial neuromodulation may include addressing each transcranial neuromodulation apparatus by a unique identifier, for example, addressing the first neuromodulation apparatus with a first unique address and the second neuromodulation apparatus with a second unique address.

As mentioned, in any of the methods described, a status indicator may be transmitted by each transcranial neuromodulation apparatus, and coordinated neuromodulation may begin for each individual after confirmation of the status indicator. For example, the first and second status indicators may be received by a third-party controller that then transmits a control signal (specifically) to the first and second transcranial neuromodulation apparatuses to apply the coordinated neuromodulation.

Also described herein are methods of securely coordinating neuromodulation of a plurality of individuals, the method comprising: establishing a first secure connection with a first transcranial neuromodulation apparatus being worn by a first individual; establishing a second secure connection with a second transcranial neuromodulation apparatus being worn by a second individual; and applying coordinated transcranial neuromodulation to both the first individual and the second individual.

For example, a method of securely coordinating neuromodulation of a plurality of individuals may comprise: establishing a first secure connection with a first transcranial neuromodulation apparatus being worn by a first individual, wherein the first transcranial neuromodulation apparatus is a self-contained, self-powered and self-adherent apparatus; establishing a second secure connection with a second transcranial neuromodulation apparatus being worn by a second individual, wherein the second transcranial neuromodulation apparatus is a self-contained, self-powered and self-adherent apparatus; and applying coordinated transcranial neuromodulation to both the first individual and the second individual.

Applying coordinated transcranial neuromodulation may include wirelessly transmitting control information controlling neuromodulation by both the first transcranial neuromodulation apparatus and the second transcranial neuromodulation apparatus. Applying coordinated transcranial neuromodulation may include applying coordinated transcranial electrical stimulation (TES), coordinated transcranial ultrasound stimulation, a combination of the two, as mentioned above, or another form of neuromodulation.

Any of the methods described herein may also include receiving confirmation from the first transcranial neuromodulation apparatus that the first individual is ready to receive neuromodulation and receiving confirmation from the second transcranial neuromodulation apparatus that the second individual is ready to receive neuromodulation before applying coordinated transcranial neuromodulation.

Applying coordinated transcranial neuromodulation may include concurrently applying the neuromodulation to both the first individual and the second individual, applying the same neuromodulation to both the first individual and the second individual, etc. For example, applying coordinated transcranial neuromodulation may include addressing the first neuromodulation apparatus with a first unique address and the second neuromodulation apparatus with a second unique apparatus.

Secure connections of each transcranial neuromodulation apparatus (e.g., with the first and second transcranial neuromodulation apparatuses) may be established by a third-party controller that transmits a control signal to the first and second transcranial neuromodulation apparatuses to apply the coordinated neuromodulation.

### BRIEF DESCRIPTION OF THE DRAWINGS

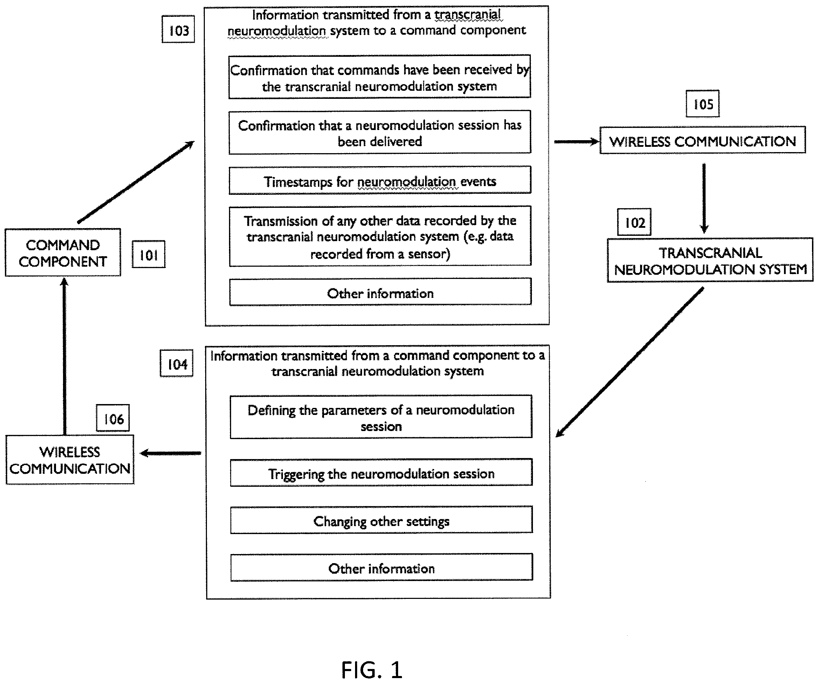

FIG. 1 is a schematic showing a configuration for wireless communication features of a transcranial neuromodulation apparatus.
FIG. 2 shows a schematic of one configuration for addressability that may be part of a transcranial neuromodulation apparatus.

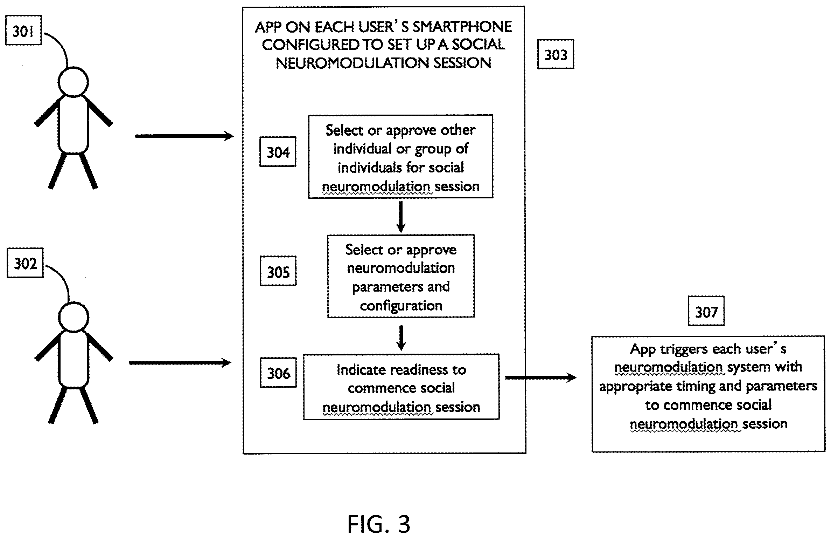

FIG. 3 is a schematic showing a workflow for coordinated neuromodulation of a group (e.g., 2 or more) of individuals.
FIG. 4A schematically illustrates one configuration for coordinated neuromodulation (e.g. by TES) in which a single neuromodulation apparatus is shared by two (or potentially more) individuals. FIGS. 4B, 4C and 4D illustrate techniques for completing the coordinated neuromodulation using an apparatus such as the one shown in FIG. 4A.
FIGS. 5A and 5B illustrate one variation of a transcranial neuromodulation apparatus configured as a lightweight, wearable and self-contained electrical stimulation (TES) apparatus including a primary unit having a secondary unit tethered by a cable. FIG. 5B illustrates the apparatus of FIG. 5A worn on an individual.
FIG. 6A is a bottom view of one variation of a transcranial neuromodulation apparatus configured to deliver ultrasound; this apparatus includes a disposable portion (applicator) and a reusable/semi-disposable portion (housing). FIG. 6B is an exploded view of the transcranial ultrasound neuromodulation apparatus shown in FIG. 6A (including the components housed within the outer housing).
FIG. 7 shows a workflow for configuring, actuating, and ending a TES session in an autonomous mode.
FIG. 8 shows components of a portable, wired TES system.
FIG. 9 shows components of a TES system that connects wirelessly to a control unit comprising a microprocessor.
FIG. 10 shows mobile computing device user interface display and functionality for connecting a wearably attached neuromodulation device to a mobile computing device caused by software stored on a non-transitory computer readable medium and executable by the mobile computing device.
FIG. 11 shows mobile computing device user interface display and functionality for selecting a wearably attached transcranial electrical stimulation device to connect to a mobile computing device caused by software stored on a non-transitory computer readable medium and executable by the mobile computing device.
FIG. 12 shows mobile computing device user interface display and functionality for indicating errors have occurred for connecting a wearably attached transcranial electrical stimulation device to a mobile computing device caused by software stored on a non-transitory computer readable medium and executable by the mobile computing device.
FIG. 13 shows mobile computing device user interface display and functionality to set up, execute, and provide feedback about a solo transcranial electrical stimulation session caused by software stored on a non-transitory computer readable medium and executable by the mobile computing device.
FIG. 14 shows mobile computing device user interface display and functionality for selecting a neuromodulatory effect to be induced by a transcranial electrical stimulation device caused by software stored on a non-transitory computer readable medium and executable by the mobile computing device.
FIG. 15 shows mobile computing device user interface display and functionality for selecting the intensity and duration of transcranial electrical stimulation caused by software stored on a non-transitory computer readable medium and executable by the mobile computing device.
FIG. 16 shows mobile computing device user interface display and functionality for instructing a user on the placement of electrodes of a transcranial electrical stimulation device caused by software stored on a non-transitory computer readable medium and executable by the mobile computing device.
FIG. 17 shows mobile computing device user interface display and functionality for controlling a transcranial electrical stimulation protocol during a neuromodulation session caused by software stored on a non-transitory computer readable medium and executable by the mobile computing device.
FIG. 18 shows mobile computing device user interface display and functionality for selecting an effect to be delivered to a user by transcranial electrical stimulation device caused by software stored on a non-transitory computer readable medium and executable by the mobile computing device.
FIG. 19 shows mobile computing device user interface display and functionality for a user to provide feedback during a transcranial electrical stimulation session caused by software stored on a non-transitory computer readable medium and executable by the mobile computing device.
FIG. 20 shows mobile computing device user interface display and functionality for a user to stop a transcranial electrical stimulation session caused by software stored on a non-transitory computer readable medium and executable by the mobile computing device.
FIG. 21 shows mobile computing device user interface display and functionality for providing retrospective data about a transcranial electrical stimulation session caused by software stored on a non-transitory computer readable medium and executable by the mobile computing device.
FIG. 22 shows mobile computing device user interface display and functionality for a user to share information about a transcranial electrical stimulation via social media caused by software stored on a non-transitory computer readable medium and executable by the mobile computing device.
FIG. 23 shows mobile computing device user interface display and functionality for a user to retrospectively provide feedback about the experience of and electrode positions for a transcranial electrical stimulation session caused by software stored on a non-transitory computer readable medium and executable by the mobile computing device.
FIG. 24 shows mobile computing device user interface display and functionality showing a historical list of transcranial electrical stimulation sessions caused by software stored on a non-transitory computer readable medium and executable by the mobile computing device.
FIG. 25 shows mobile computing device user interface display and functionality for selecting a previously experienced transcranial electrical stimulation session and triggering it to repeat caused by software stored on a non-transitory computer readable medium and executable by the mobile computing device.
FIG. 26 shows mobile computing device user interface display and functionality for providing descriptive information and error signal notices caused by software stored on a non-transitory computer readable medium and executable by the mobile computing device.
FIG. 27 shows mobile computing device user interface display and functionality to set up (as a host), execute, and provide feedback about a group transcranial electrical stimulation session caused by software stored on a non-transitory computer readable medium and executable by the mobile computing device.
FIG. 28 shows mobile computing device user interface display and functionality to set up (as a participant), execute, and provide feedback about a group transcranial electrical stimulation session caused by software stored on a non-transitory computer readable medium and executable by the mobile computing device.
FIG. 29 shows mobile computing device user interface display and functionality to wait for participants to join a group transcranial electrical stimulation session caused by software stored on a non-transitory computer readable medium and executable by the mobile computing device.
FIG. 30 shows mobile computing device user interface display and functionality for a user in a group transcranial electrical stimulation session to indicate they are ready to join the group session caused by software stored on a non-transitory computer readable medium and executable by the mobile computing device.
FIG. 31 shows mobile computing device user interface display and functionality for the host of a group transcranial electrical stimulation session to send an effect to the transcranial electrical stimulation device worn by a participant in a group transcranial electrical stimulation session caused by software stored on a non-transitory computer readable medium and executable by the mobile computing device.

### DETAILED DESCRIPTION

In general, described herein are networkable transcranial neuromodulation apparatuses adapted for safely and effectively applying neuromodulation that is coordinated with a group of other individuals, as well as methods for securely and effectively applying coordinated neuromodulation. The apparatuses described herein may include systems, devices, and the like. Any of the features described herein may be included in single devices (single neuromodulation devices) that may be operated as part of a network, or that may operate separately without being networked (e.g., "solo"); in some variations these apparatuses may be switched between solo operation and networked operation, as described in detail below.

For example, described herein are systems for transcranial neuromodulation configured to modulate neuronal activity, including but not limited to systems for transdermal electrical stimulation (TES, including transcranial electrical stimulation) and transcranial ultrasound neuromodulation. A transcranial neuromodulation system may be self-adhesive to the head, self-powered (e.g. by a battery), and self-contained (i.e. containing all components in a single housing) and referred to as a 'puck'. Transcranial ultrasound neuromodulation pucks may also be self-coupling. Transcranial neuromodulation pucks may also be disposable or semi-disposable. The apparatuses described herein may deliver non-invasive neuromodulation transdermally; as mentioned, this neuromodulation may be transcranial. Unless the context indicates otherwise, transcranial delivery is one type of transdermal neuromodulation, and any of the transcranial configurations may be generally applied transdermally.

The apparatuses described herein may be configured as transcranial electrical stimulator (TES) apparatuses. The cognitive effect(s) may arise from one or a combination of stimulation effects, including stimulation of nerves (e.g., cranial nerves) and/or brain cells. Any appropriate electrical stimulation may be applied by the apparatus to provoke the desired cognitive effect. For example, a controller may be configured to cause alternating current, direct current, or a combination of alternating and direct current between the first and second electrodes.

The term 'transcranial neuromodulation system' may refer to any system configured for transcranial neuromodulation including, but not limited to: transcranial ultrasound neuromodulation, transdermal electrical stimulation (TES, including transcranial electrical stimulation), combined TES and transcranial ultrasound neuromodulation, or another noninvasive form of neuromodulation. The term 'neuromodulation puck' may refer to any puck configured for transcranial neuromodulation using a technique including, but not limited to: transcranial ultrasound neuromodulation, transdermal electrical stimulation (TES, including transcranial electrical stimulation), combined TES and transcranial ultrasound neuromodulation, or another noninvasive form of neuromodulation. The transcranial neuromodulation pucks (also referred to herein as 'neuromodulation pucks') and other transcranial neuromodulation systems (also referred to herein as 'neuromodulation systems') described herein may have one or more features selected from the group comprising: addressability, personalization, wireless communication, security, privacy, remote triggering, synchronization for social aspects of neuromodulation among two or more individuals, sensors for recording brain activity, sensors for measuring physiology, and sensors for triggering on the basis of location, context, or proximity to another user. These features are beneficial for neuromodulation systems useful in daily life. Each listed feature will be described in detail in this specification.

Transcranial electrical stimulation (TES) is advantageous for modulating brain activity and cognitive function in man. Neurons and other cells in the brain are electrically active, so stimulation using electric fields is an effective strategy for modulating brain function. In various embodiments of the invention, the effect of neuromodulation induced by TES is one or more of inhibition, excitation, or modulation of neuronal activity. A TES neuromodulation puck (also referred to herein as a 'TES puck') or other TES neuromodulation system (also referred to herein as a 'TES apparatus' or 'TES system') may comprises two or more electrodes for delivering electrical stimulation and is configured with appropriate hardware and software (e.g. firmware) for controlling the intensity, duration, and other parameters of electrical stimulation. In some embodiments of the invention, a TES puck incorporates one or more features selected from the group including, but not limited to: dry electrodes, multiple electrodes configured as a current source and/or current sink, arrays of electrodes, and electrode arrays configured for targeted TES based on finite element modeling (FEM). A TES puck or other TES system may be configured for one or more stimulation regime selected from the group consisting of: transcranial alternating current stimulation (tACS), transcranial direct current stimulation (tDCS), and transcranial random noise stimulation (tRNS). A TES puck or other TES system may incorporate an array of TES electrodes.

Transcranial ultrasound neuromodulation is useful for affecting brain function by activating, inhibiting, or modulating neuronal activity. A transcranial ultrasound neuromodulation system comprises at least one ultrasound transducer and appropriate hardware and software (e.g. firmware) for controlling the intensity, duration, pulsing, acoustic frequency, and other parameters of ultrasound energy delivered. A transcranial ultrasound neuromodulation puck or other transcranial ultrasound neuromodulation system may incorporate one or more features selected from the group consisting of: lenses for focusing ultrasound energy, an array (e.g. a phased array) of ultrasound transducers, self-coupled (i.e. incorporating an acoustic couplant material to form a low acoustic impedance contact with the head), a solid acoustic couplant, and one or more capacitive micromachined ultrasound transducers (CMUTs).

Pucks or other systems that combine TES and transcranial ultrasound neuromodulation deliver both ultrasound energy and electrical stimulation to the head of a user and are beneficial embodiments of the present invention. In certain embodiments, pucks that combine TES and transcranial ultrasound neuromodulation delivery need not provide both TES and transcranial ultrasound neuromodulation in a single session. In a single session, pucks with the ability to provide both TES and transcranial ultrasound neuromodulation may provide TES neuromodulation, transcranial ultrasound neuromodulation, or any combination thereof. Configuration of which neuromodulation techniques applied may be provided by control components located in/on the puck or remotely as connected wirelessly or through a wired connection.

Neuromodulation induced by a transcranial neuromodulation puck or other transcranial neuromodulation system described herein can be configured to affect one or more brain regions that mediate sensory experience, motor performance, learning, memory, and the formation of ideas and thoughts, as well as states of emotion, physiological arousal, sexual arousal, attention, creativity, relaxation, empathy, connectedness, and other cognitive states. In embodiments of the invention, the effect of neuromodulation is detected by one or more method chosen from the group including, but not limited to: subjectively by the recipient as a perception, movement, concept, instruction, other symbolic communication by modifying the recipient's cognitive, emotional, physiological, attentional, motivational, or other cognitive state; (ii) through physiological measurement of brain activity by one or a plurality of: electroencephalography (EEG), magnetoencephalography (MEG), functional magnetic resonance imaging (fMRI), functional near-infrared spectroscopy (fNIRS), positron emission tomography (PET), single-photon emission computed tomography (SPECT), computed tomography (CT), functional tissue pulsatility imaging (fTPI), xenon 133 imaging, magnetic resonance spectroscopy (MRS), or other techniques for measuring brain activity known to one skilled in the art; and (iii) by making a physiological measurement of the body such as by electromyogram (EMG), galvanic skin response (GSR), electrocardiogram (EKG), pulse oximetry (e.g. photoplethysmography), heart rate, blood pressure, respiration rate, pupil dilation, eye movement, gaze direction, and other physiological measurement.

In any of the apparatuses described herein, a transcranial neuromodulation apparatus (e.g., a neuromodulation puck) may typically include components for wireless communication to and from a command center that can take the form of a system selected from the group including, but not limited to: a smartphone or tablet; laptop or desktop computer; remote control communicating by infrared light signals or a wireless communication protocol; or remote computer (e.g. server) communicating via the Internet and/or cellular data protocols.

Wireless communication to a transcranial neuromodulation system is configurable for command component 101 (e.g., remote computing device) to send information to and receive information from a transcranial neuromodulation system 102 or component (e.g., puck). Information transmitted wirelessly 105 from a command component to a transcranial neuromodulation system includes, but is not limited to: 103 defining the parameters of a neuromodulation session, triggering the neuromodulation session, and changing other settings. Information transmitted wirelessly 106 from a transcranial neuromodulation system to a command component includes, but is not limited to: 104 confirmation that commands have been received by the transcranial neuromodulation system, confirmation that a neuromodulation session has been delivered, timestamps for neuromodulation events, and transmission of any other data recorded by the transcranial neuromodulation system (e.g. data recorded from a sensor). Communication from a controller wirelessly to a neuromodulation stimulator system can include information to control the intensity, waveform, and timing of energy delivered to a subject to induce neuromodulation, including, but not limited to: information about a waveform to be delivered such as a stimulation frequency, a pulse repetition frequency, a duty cycle, a direct current offset, an alternating current shape, a frequency modulation, and an amplitude modulation; information about a when to start, pause, re-start, and end a stimulation session; information about a peak intensity; information about an average intensity; information about triggers for starting, pausing, or ending stimulation based on a user's location, physiological state, cognitive state, or control by another third-party controller. Any of the communications to/from the apparatuses may be encrypted or otherwise secured. For example, the apparatus may be configured to require a security key or may encrypt some or all information transmitted using a security key that is shared with a verified receiver.

A wireless signal received by a transcranial neuromodulation system may be a trigger to instruct neuromodulation to commence, end, or change, and the transcranial neuromodulation system has one or more fixed transcranial neuromodulation protocols pre-configured that are delivered upon receiving the trigger signal.

A communicated signal may include information about the transcranial neuromodulation protocol to be delivered. In TES embodiments, the communicated signal about the transcranial neuromodulation protocol to be delivered is selected from the group including, but not limited to: onset time, intensity, duration, number of repeats, ramping of a parameter, one or more frequencies (for tACS or pulsed operation), ramp frequency characteristics (for tACS), noise characteristics (for tRNS), or other feature that defines a time-varying electrical stimulation protocol. In transcranial ultrasound neuromodulation embodiments of the invention, the communicated signal about the transcranial neuromodulation protocol to be delivered is selected from the group including, but not limited to: onset time, intensity, duration, number of repeats, acoustic frequency characteristics, pulse duration, pulse repetition frequency, and ramping of a parameter.

The apparatuses described herein can be designed to communicate with other devices in a wireless fashion. Communication may be made with devices and controllers onboard or remotely located using methods known in the art, including but not limited to, RF, WIFI, WiMax, Bluetooth, BLE, UHF, NHF, GSM, CDMA, LAN, WAN, or another wireless protocol. Pulsed infrared light as transmitted for instance by a remote control is an additional wireless form of communication for communicating to a neuromodulation system or neuromodulation puck. Near Field Communication (NFC) is another useful technique for communicating with a neuromodulation system or neuromodulation puck. One of ordinary skill in the art would appreciate that there are numerous wireless communication protocols that could be utilized with embodiments of the present invention, and embodiments of the present invention are contemplated for use with any wireless communication protocol.

A neuromodulation puck or other neuromodulation system may return a confirmation signal concerning whether a scheduled neuromodulation session was delivered. A confirmation signal may be transmitted via the Internet to a remote server. The neuromodulation confirmation data may be used to provide feedback to one or more of the group including, but not limited to: the user, a third party, a user's friend, a doctor, a teacher, or one or more members of the public who receive data that is broadcast widely through a blog posting, Facebook post, tweet, or other form of social communication or web publishing.

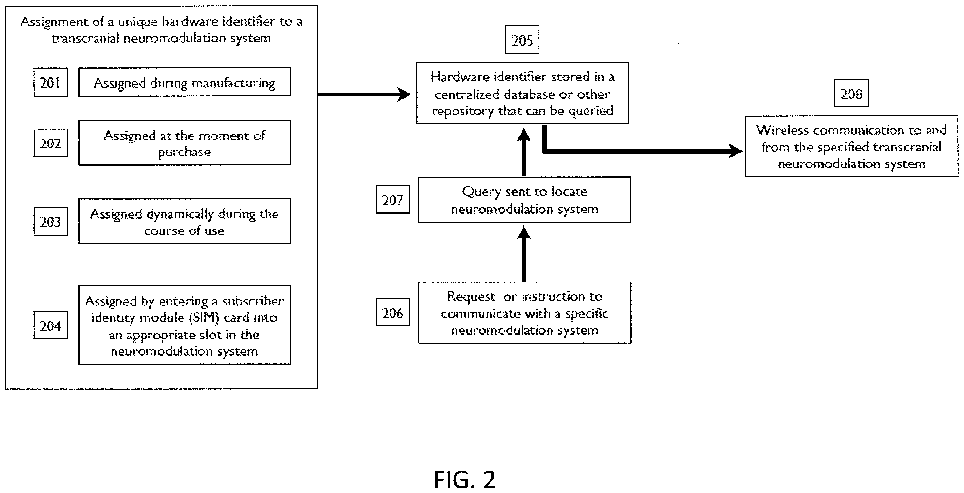

A neuromodulation system may be addressable, as illustrated in FIG. 2. Addressability is an advantageous feature, because it identifies a particular instance of a neuromodulation system from other instances so that a particular neuromodulation system can be located, identified, and communicated with in a targeted manner. A neuromodulation puck is an example of a neuromodulation system useful for addressability, because pucks are modular and can be configured to operate independently.

In order to be addressable, a neuromodulation system may require a unique identifier and a communications protocol for broadcasting and/or querying the hardware identifier. Addressable systems are well known in the art but have not been previously considered in the context of neuromodulation devices. The Internet Protocol address (IP address) and Media Access Control address (MAC address) systems are examples of frameworks for addressability.

A neuromodulation system may be assigned a hardware identifier during the manufacturing process 201, similar to a MAC address for an Ethernet card. Alternatively, a neuromodulation system may be assigned a hardware identifier at the moment of purchase 202, similar to a system for activating a gift card at the time of purchase. Or, a neuromodulation system may be assigned an identifier dynamically during the course of use 203, similar to a dynamic IP address. Assignment of an identifier dynamically may be handled, for instance via use of the dynamic host configuration protocol (DHCP) provided by standard wireless and network routers. Identifiers may be given to the neuromodulation system through use of a third party component, such as through entering a subscriber identity module (SIM) card into an appropriate slot in the neuromodulation system 204.

A centralized database or other repository 205 stores hardware identifiers for neuromodulation units and is configured to be queried 207 by approved systems in order to communicate wirelessly 208 to and from the specified transcranial neuromodulation unit, for instance upon receiving a request or instruction to communicate with a specific neuromodulation system 206.

Any system for uniquely identifying a neuromodulation system known to one skilled in the art of creating hardware identifier systems can be used.

A neuromodulation system may be configured to be secure and private. Security may be an important neuromodulation system feature to ensure that it is not remotely triggered against the wishes of a user and to maintain all data stored locally on the neuromodulation system and transmitted to and from the neuromodulation system private according to the wishes of the user. Security and privacy features have not previously been considered for neuromodulation systems. A neuromodulation puck is an embodiment of a neuromodulation apparatus that is advantageously configured for security and/or privacy, because pucks are modular and can be configured to send and receive communication wirelessly.

Encryption of data stored on a neuromodulation system and transmitted to and from the neuromodulation system is an advantageous feature that improves the security and privacy of user data. Users may not want information about the number, duration, intensity, or target of their neuromodulation sessions to be shared or public. Similarly, some embodiments of the invention that record brain activity or otherwise measure physiology will generate data that users also may not want to share or make public.

In some embodiments, a neuromodulation system requires that a user 'log in' to the neuromodulation system by identifying themselves as a registered or approved user of the neuromodulation system prior to receiving access to any data stored in or functionality of the neuromodulation system. Neuromodulation pucks are embodiments of neuromodulation systems that can be configured to require a user to 'log in'. In various embodiments, a neuromodulation system can incorporate one or more security systems chosen from the group including, but not limited to: an alphanumeric password entered on a user interface component of the system; a temporal pattern of button presses on a single button; a retina scan by having the user place their eye in front of a camera incorporated in the system; a fingerprint scan achieved with appropriate hardware components of the system; a spoken password recorded by a microphone on the system and processed to confirm a user's identity; an electroencephalogram pattern recorded by one or more pairs of electrodes contained in the system; a near field communication signal or other proximity based signal (e.g., key fob) received from a device previously linked or otherwise approved; and a password entered on a third-party device (e.g. smartphone, tablet, or computer) wirelessly configured to communicate with the system.

A neuromodulation system may querry a database stored on a remote server to determine whether a password, biometric identifier, or other identification input is correct for a particular user.

A neuromodulation system may utilize secured wireless transmission protocols and communication and cryptographic protocols to further secure data transmission. Security protocols used for transmission of secured data and communications may include, but are not limited to: Transport Layer Security (TLS), Secure Socket Layer (SSL), public-key and/or private-key cryptographic protocols, Wireless Equivalent Protocol (WEP), Wi-Fi Protected Access (WPA or WPA2) protocols, or any combination thereof. One skilled in the art of data access, security, and control protocols will appreciate that there are numerous security and transmission protocols that could be used with embodiments of the present invention, and embodiments of the present invention are contemplated for use with any appropriate security and transmission protocols.

A neuromodulation system may be configured to 'lock-out' under circumstances chosen from the list including but not limited to: a fixed period of time after a previous successful login; a fixed period of time after a neuromodulation session ends; a fixed period of time after a neuromodulation session begins; in response to a user interface action by a user causing password lock to go into effect; when a neuromodulation system is removed from the user's head; and remotely through a wireless connection.

A neuromodulation system may be configured to allow a user to control permissions for third party access. In various embodiments, third party access refers to one or more function chosen from the group including, but not limited to: access to data transmitted to a neuromodulation system; access to data transmitted from a neuromodulation system; access to data stored on a neuromodulation system; access to a neuromodulation system data repository or database stored on a remote server and accessed via the Internet; control of triggering the onset of neuromodulation; control of triggering the offset of neuromodulation; control of the settings for neuromodulation; access granted to a third party service from a user (e.g. permitting data to be automatically posted on Facebook); and control of 'lock-out' of neuromodulation system so that neuromodulation cannot be delivered.

In general, any of the apparatuses and method described herein may be configured so that an individual may control third party access. Alternatively or additionally, control of third party access is managed through a user interface on a neuromodulation system or through an app or website accessed on a separate device such as computer, smartphone, or tablet that transmits information about third party access settings to the neuromodulation system. Privacy settings can be defined generally to control third party access for any individual, entity, or system. For example, a user may permit access to their data so that their neuromodulation sessions can be shared via a social network. Privacy settings can also be defined for a specific individual, entity, or system. For example, a user may permit a significant other or medical professional to control the triggering of their neuromodulation sessions.

Any of the apparatuses described herein configured to permit third party access may include a system for prioritizing instructions for controlling the neuromodulation system when conflicting signals are received. The neuromodulation system control system can be configured for a user to manually select the order of priority for neuromodulation system control signals. Alternatively, the system can automatically determine a priority based on the source of the signal (i.e. the user herself is highest priority; the user's identified family members are second level priority; the user's identified friends are a third level priority; automated systems are a fourth level priority). One skilled in the art of third party access controls will appreciate that priority levels can be automatically assigned according to any acceptable criteria.

A neuromodulation system may be pre-configured with security and privacy settings. Disposable and semi-disposable neuromodulation pucks with pre-configured security and privacy settings are advantageous, because they permit a user to select a system with security and privacy settings to suit their needs at a particular time. For example, a user normally uses a neuromodulation system with permissive privacy settings, but selects a disposable system with stricter privacy settings for a vacation when they do not want third parties to access their data or their neuromodulation system.

A useful privacy feature may include configurability for the functional equivalent of 'do not disturb' or 'out of office' for times when a user does not want to receive a triggered neuromodulation protocol from someone else. Another useful privacy feature may include configurability to discretely reject a connection request.

Third party access may be achieved through an application programming interface (API). A neuromodulation apparatus may be configurable to lock out stimulation in unsafe situations such as driving, certain forms of work, or when a user is under the influence of drugs or alcohol.

A neuromodulation apparatus may provide parents or other care providers the ability to control neuromodulation delivered to a minor or other individual who is not able to make their own decisions (e.g., an adult with intellectual disability or elderly person with dementia). For a neuromodulation system that has a non-disposable and disposable portion, the parental controls would most advantageously be on the non-disposable portion. Alternatively, parents can purchase disposable units that have different settings amenable for use in children and cannot be altered.

A neuromodulation system may be configured for synchronization with other neuromodulation systems wearably attached to one or more other users. Herein, we refer to paired, group, and social applications of neuromodulation systems as "social neuromodulation". A neuromodulation puck is an embodiment of a neuromodulation system that is beneficial for social neuromodulation. Particularly advantageous social neuromodulation applications are configured for use by a pair of individuals or a small group of individuals.

Neuromodulation systems configured for social neuromodulation may be advantageous for paired neuromodulation. Two individuals, each wearing at least one neuromodulation system coordinate a neuromodulation session. Paired neuromodulation can be configured to occur between two individuals in each other's presence or two individuals at a physical distance. In some embodiments of paired neuromodulation, the users' neuromodulation systems are configured to induce a change in cognitive state synchronously or close together in time. Paired neuromodulation systems are configured to require that each user first approve the paired neuromodulation session, then triggers each user's neuromodulation system to begin neuromodulation.

In a one example of a social neuromodulation session, users 301, 302 access 'app' on a smartphone or tablet 303 to: (1) select or approve other individual or group of individuals for social neuromodulation session 304; (2) select or approve neuromodulation parameters and configuration 305; and (3) indicate readiness to commence social neuromodulation 306. Once all users in a social neuromodulation session have indicated their readiness to commence the social neuromodulation session, the app and related systems (e.g. on a remote server accessed by the app via the Internet) triggers each user's neuromodulation system with appropriate timing and parameters to commence social neuromodulation session 307.

A neuromodulation apparatus can be configured for social neuromodulation by more than two users, herein referred to as group social neuromodulation. The components, configurations, and benefits of group social neuromodulation are similar as those for paired neuromodulation, but require additional controls to confirm that all members of the group social neuromodulation session approve it and have a neuromodulation system wearably attached and ready to deliver neuromodulation. In an aspect of an embodiment of social neuromodulation, a user can pause a neuromodulation session being received as part of a pair or group, then resume the neuromodulation at a desired future time.

A neuromodulation system can be configured to induce any shared cognitive effect for social neuromodulation. For example, paired neuromodulation can be configured to induce states of calm, energy, flow, or creativity. Two or more users meditating together would benefit from a shared state of calm. Two or more users brainstorming patent content would benefit from a shared state of flow and/or creativity. Two or more users at a dance club would benefit from a shared state of energy.

A paired social neuromodulation session may be configured so that one individual receives neuromodulation intended for a particular modification of cognitive state, while another individual receives neuromodulation intended for a different modification of cognitive state. A neuromodulation system designed in this manner can be used to increase confidence in one individual, and make another individual receptive for a conversation (e.g. a conversation in the context of counseling, tutoring, or lecturing). In another embodiment of social neuromodulation, among two people working together as a brainstorming team, one receives neuromodulation to enhance creative thinking while another receives a different form of neuromodulation for more ordered rational thinking. In another embodiment of social neuromodulation, individuals working as a group in a stressful situation such as a surgical team, first responders, or soldiers receive concurrent neuromodulation to induce a state of calm and/or reduced anxiety.

In some embodiments of group social neuromodulation, one or more parameters of neuromodulation is different between members of the group. Differences in neuromodulation parameters would occur if different users have different neuromodulation devices or if personalized settings for each user define parameters optimized for that user. In an embodiment of the invention, a desired endpoint of neuromodulation in a social neuromodulation session is defined as a brain state or cognitive state, and each individual receives an appropriate neuromodulation input to achieve that desired endpoint. In this manner, each individual may receive neuromodulation that differs in timing, intensity, targeting, or another parameter, each stimulation protocol intended to achieve a similar change or endpoint of neural activity or cognitive function.

In some embodiments of group social neuromodulation, two or more individuals receive neuromodulation that causes a synchronized coordination of their brain states. For instance, the relative power of brain rhythms (e.g. alpha or gamma) can be coordinated. Each individual may require different neuromodulation stimulation parameters and/or targets to achieve a common brain state. Thus, advantageous embodiments for coordinated brain states during a social neuromodulation session incorporate a brain recording or other physiological measurement to estimate a current brain state, then are configured to deliver neuromodulation with appropriate parameters to shift from that current brain state to the intended coordinated brain state.

In an embodiment of the invention, a user broadcasts that they are interested in a social neuromodulation session. This intent can be communicated by a custom app, web posting, or other form of distributed (likely Internet-enabled) communication. A social neuromodulation session optionally occurs when users are in a common location or, alternatively, when they are remote from each other. In an example of social neuromodulation, communication through a social media network such as Facebook, Twitter, or Instagram is used to coordinate the social neuromodulation session among users.

In a system configured for TES neuromodulation, TES current flows between two or more users. Herein, we refer to this example as a "mutual TES". A core feature of mutual TES is that the current sink and current source are on different users. The two or more users must touch in an electrically conductive way for current to flow into and between them. Electrically conductive touch can occur by mutually touching moist or wet skin anywhere on the body for instance by touching lips or by having one or more of the users hold an electrically conductive pad that each user touches (e.g. via a handshake). In an example of mutual TES, there is a single current source (or current sink) and many current sinks (or current sources). An example configuration for this embodiment requires that everyone sits in a circle and touches something conductive at the same time. In this example, the experience of any one individual is affected by the presence or absence of another individual in the circuit.

The social neuromodulation examples illustrated above generally illustrate coordinated group neuromodulation. Elements from any of these examples may be adapted for use in other (even more general) variations.

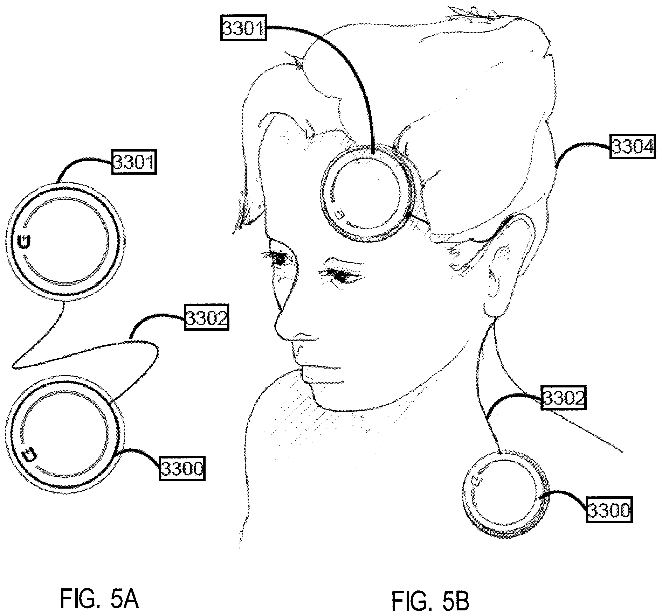

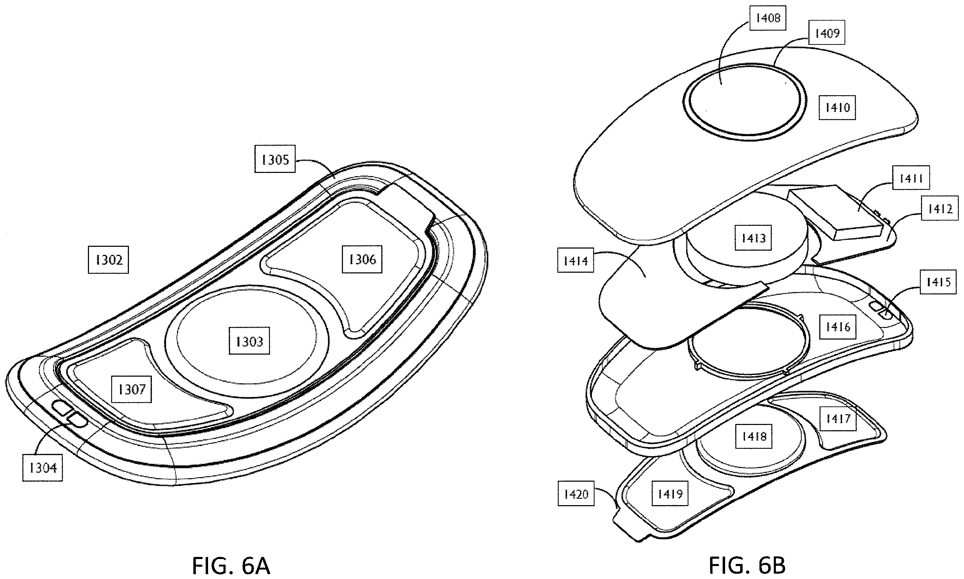

FIGS. 5A-5B and 6A-6B illustrate examples of transdermal (e.g., transcranial) neuromodulation apparatuses that may be used. For example, FIGS. 5A and 5B illustrate one example of a lightweight, wearable and self-contained transcranial neuromodulation apparatus configured for electrical stimulation (TES). In this example, the apparatus includes a primary unit 3300 housing a power source, processor/controller, and wireless communication module. The outer housing of the apparatus includes an indicator 3305 which can be illuminated when the device is on and ready to operate; an LED light may indicate status (e.g., on/off, transmit/receive, etc.). The primary unit also includes an electrode that can be placed in contact with the subject's skin, as illustrated in FIG. 5B. A secondary unit 3301 is connected to the primary unit by a cable 3302. The secondary unit also includes an electrode and can be adhesively attached to the subject. In this example, the primary unit 3300 is connected to the subject's neck/shoulder region and the secondary unit 3301 is independently positioned and adhesively connected to the subject's head, as illustrated in FIG. 5B. The positions of the primary and secondary units may be reversed. Other examples of such apparatuses maybe found, for example, in US patent application number 14/091,121, filed on 11/26/2013.

FIGS. 6A-6B illustrate one variation of a transcranial neuromodulation apparatus that is configured to apply ultrasound. For example, FIG. 6A shows a line drawing 1302 of the top of a transcranial ultrasound neuromodulation puck, including a gel interface area 1303, adhesive areas 1306, 1307, charger contacts 1304, and housing 1305.

FIGS. 6B shows an exploded view of the apparatus of FIG. 6A from a top view. The top view shows disposable portion 1420, adhesive areas 1419, 1417, solid acoustic couplant puck 1418, charger contacts 1415, housing 1416, 1410, printed circuit boards 1412, 1414, ultrasound transducer 1413, battery 1411, on/off button 1408, and LED indicator ring 1409 to indicate when ultrasound is being delivered to the user. Other examples of transcranial neuromodulation apparatuses configured to deliver ultrasound may be found, for example, in PCT/US2014/016178, filed on February 13, 2014.

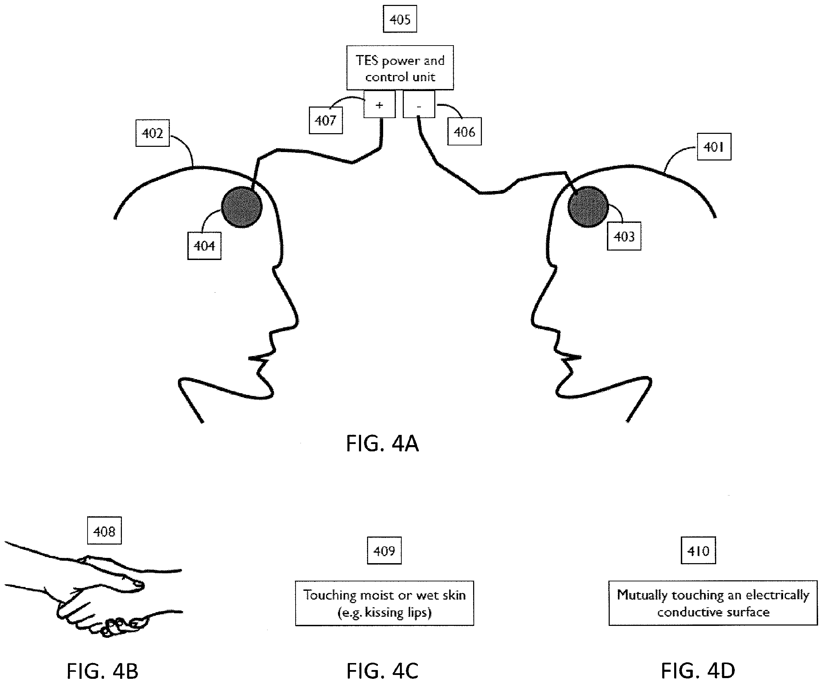

FIG. 4A shows a sample configuration for mutual TES. Individuals 401, 402 each wear single electrodes 403, 404 that connect to single TES power and control unit 405. Either electrode can be plugged into the power and control unit anode 407 or cathode 406 connections. No current is passed into either electrode until a return electrical path is creating by the two individuals. FIGS. 4B-4D illustrate ways to create a return path to provide mutual TES using a system such as the one shown in FIG. 4A. For example, a return path can be created: (as shown in FIG. 4B) by touching a part of the skin that is not a low impedance electrical conductor but is made conductive by having an electrically conductive pad, gel, or other material that is electrically conductive between the two individuals 408; (as shown in FIG. 4C) by touching moist or wet skin (e.g. kissing lips) 409; and (as shown in FIG. 4D) by mutually touching an electrically conductive surface 410.

An embodiment of a neuromodulation system for group social neuromodulation is configured to have one controller of neuromodulation and two or more recipients of neuromodulation, where the number of recipients is optionally greater than 3 recipients, greater than 4 recipients, greater than 5 recipients, greater than 10 recipients, greater than 50 recipients, greater than 100 recipients, greater than 250 recipients, greater than 1000 recipients, greater than 10000 recipients, greater than 100000, or a larger number of recipients. A neuromodulation system for group social neuromodulation configured in this manner would be entertaining and socially interesting. An example of this embodiment is a concert where each member of the audience receives a neuromodulation puck as they enter the venue and are instructed to put it on at a particular time in the performance. A transmitter box and antenna is configured to transmit wirelessly to each neuromodulation puck and induce a shared change in cognitive function (or a set of different effects of cognitive function among the audience members). Not all members of the neuromodulation group need receive the same form of neuromodulation at the same time.

In another configuration of the system for group social neuromodulation, the system is configured so that no single top-down controller is required. Rather, all members of the group indicate that they are ready to begin a neuromodulation session through a user interface on their neuromodulation puck; through a user interface on an app or other software on an external device wireless communicable to the neuromodulation puck; or by accessing a website (e.g. through a QR code). Once group members have indicated they are ready to join the group neuromodulation session, the system automatically triggers the neuromodulation session. The workflow is similar to how a conference call is initiated. Group social neuromodulation is advantageous for multi-player games (i.e. a game can be designed to differentially alter the states of the players depending on their interactions in the game).

For users who select permissive privacy settings, other individuals can 'follow' the user and receive updates about neuromodulation that the user has received, as well as related data generated by their neuromodulation system. In an embodiment, a second user can configure his neuromodulation system to trigger a similar neuromodulation session at the same time as a first user who has configured her system to broadcast or otherwise make public a neuromodulation session she has commenced or will commence. In this manner, the second user can share in the cognitive state of a famous athlete, entertainer, or other individual. Such a system would optionally include a push notification to the second user that a session will begin soon or at a defined time and where on the head a neuromodulation system should be placed so that the second user is prepared for a shared neuromodulation session. For neuromodulation 'following', the followed individual optionally employs an interface enabling them to indicate when and what type of neuromodulation they intend to deliver.

A neuromodulation apparatus may be shared between two or more individuals, as described and illustrated above. A neuromodulation puck is an example of a neuromodulation system that is easily shared due to its small size and portability. In some examples with neuromodulation system sharing, a neuromodulation system is configured with multiple 'logins' that can be accessed by separate passwords, different biometric profiles, or another method for distinguishing users. This type of system is useful for family members, friends, teammates, classmates, roommates, or other groups of individuals who have repeated interactions. In an alternative system with neuromodulation system sharing, the user for a particular piece of hardware is changed remotely. A technician, service, or other third party activates a neuromodulation system remotely for a user. Car sharing services such as Zipcar use a similar system whereby company technicians can remotely unlock a car for a particular user.

In alternative examples with neuromodulation system sharing, a third party or technician pre-configures a neuromodulation system for use by a new user and no personal data is stored on the system. This type of system is useful in rental contexts, at a neuromodulation cafe, library, school, place of work, spa, mall, clinic, or other shared or public space.

In other examples with neuromodulation system sharing, a user lends their puck to a second user and provides them with guest access which keeps the first user's information secure and optionally is configured to disable or enable certain features of the neuromodulation system. Another aspect of the system is the ability to share configurations (e.g. placement of neuromodulation systems on the head) and parameters (of neuromodulation energy delivered) with one or more other users chosen from the group including, but not limited to: a user's friend, a doctor, a teacher, or one or more members of the public who receive data that is broadcast widely through a blog posting, Facebook post, tweet, or other form of social communication or web publishing.

A neuromodulation puck may be configured to be triggered remotely or automatically. A neuromodulation puck is an example of a neuromodulation system for which remote or automated triggering is useful due to the portability of pucks.

Controlling neuromodulation according to a user's location is a beneficial feature for automatically starting, ending, or modifying the parameters of neuromodulation. One or more systems for determining the location of a user is chosen including, but not limited to: RFID, OPS, Wi-Fi network, IP address, 'check-in' by the user with a service such as Foursquare or Yelp indicating their presence at a particular location, facial recognition from a camera at a known location, or another system for determining a user's location.

A user can pre-configure their neuromodulation system to be controlled based on location or select a privacy setting that permits a third party to control the user's neuromodulation system based on the user's location. Location-based remote control of a neuromodulation system can affect neuromodulation upon a user being present at a particular location by one or more of the events chosen from the group including, but not limited to: immediate triggering of neuromodulation; delayed triggering of neuromodulation at a specific latency; delayed triggering of neuromodulation randomly within a window of time; delayed triggering until one or more other users is present at the same location, then trigger immediately, at a defined latency, or randomly within a window of time; continuous neuromodulation while a user is present at the location; and immediately triggering neuromodulation when a user leaves a location, at a defined latency after leaving the location, or randomly within a window of time after leaving the location. Controlling neuromodulation according to a user's proximity to a second user is a beneficial feature for automatically starting, ending, or modifying the parameters of neuromodulation.

Any of the neuromodulation apparatuses described herein may be configured for scheduling a neuromodulation session. A neuromodulation puck is an example of a neuromodulation system for which scheduling is advantageous due to the portability and autonomous function of pucks.

Scheduling a neuromodulation event may include transmission of one or more of the following types of information to a neuromodulation system or to a server communicably interfaced with a neuromodulation system chosen from the group consisting of: onset of neuromodulation, offset of neuromodulation, duration of neuromodulation, intensity, target, or other parameter of neuromodulation. In examples of a neuromodulation system configured for scheduling, scheduling is achieved through one or more of the group including, but not limited to: a user interface on the device itself, like scheduling an alarm on a watch or smartphone; by a custom web or mobile interface that provides a user interface for scheduling; by integrating with an API for an Internet calendar such as Google calendar; and through any other web service that enables a user to control (schedule) when a signal is sent to a third party server. Alternatively, scheduling can be controlled by a third party if the user has given appropriate permissions. For example, a corporate assistant can schedule neuromodulation to focus a coworker before an important meeting. In another example, a tutor or teacher schedules a neuromodulation session for improved attention to occur during a study period for a student. In another example, a coach schedules a neuromodulation session to begin immediately before an athletic practice so that the athlete's brain is primed for motor learning.

Any of the transcranial neuromodulation apparatuses described herein may further comprise one or more sensors. A neuromodulation puck is an embodiment of a neuromodulation system for which including sensors is advantageous because autonomous function of the puck can be controlled based on data recorded by the one or more sensors. A neuromodulation apparatus may incorporate or receive data from one or more sensors that determine the user's location or proximity to another user. A neuromodulation system may incorporate or receive data from one or more sensors for recording brain activity. In this embodiment of the invention, brain activity is measured using one or more techniques chosen from the group consisting of: electroencephalography (EEG), magnetoencephalography (MEG), functional magnetic resonance imaging (fMRI), functional near-infrared spectroscopy (fNIRS), positron emission tomography (PET), single-photon emission computed tomography (SPECT), computed tomography (CT), functional tissue pulsatility imaging (ITPI), xenon 133 imaging, magnetic resonance spectroscopy (MRS), or other techniques for measuring brain activity known to one skilled in the art.

A neuromodulation system may incorporate or receive data from one or more sensors for measuring physiology. In this embodiment of the invention, physiology is one or more chosen from the group consisting of: electromyogram (EMG), galvanic skin response (GSR), electrocardiogram (EKG), pulse oximetry (e.g. photoplethysmography), heart rate, blood pressure, respiration rate, pupil dilation, eye movement, gaze direction, or other physiological measurement known to one skilled in the art.

Recordings of brain activity or other physiological measurements may be processed on the device and can be used to alter the onset, duration, or other parameters of neuromodulation. Raw and/or processed recordings of brain activity or other physiological measurements are optionally stored locally on the neuromodulation system and optionally transmitted wirelessly to a base station, computer, smartphone, or tablet, or transmitted to a remote server via the Internet. Data about brain recordings or other physiological measurements before, during, and after neuromodulation are optionally shared by the user to a third party individual or service based on the user's selected privacy and sharing settings.

Sensors may also be advantageous for scheduling neuromodulation. For instance, neuromodulation can be automatically scheduled based on a stress level being exceeded. Alternatively, a neuromodulation protocol for arousing a subject can be scheduled based on the amount of sleep (or a specified sleep state) a user experiences as measured by an EEG sensor component of the neuromodulation system.

A neuromodulation puck or other neuromodulation system may include a computer memory component so that a signal can be received and, for instance, a future scheduled neuromodulation session can be saved. Even if there is no data connection to the device in the intervening period, the scheduled session is queued to proceed at the appropriate time.

A neuromodulation puck or other neuromodulation system may include a second battery to provide uninterrupted power to a clock and/or other electronic components. Having a secondary source of power is useful for maintaining settings, scheduling future neuromodulation sessions, and other clock or memory applications.

A neuromodulation puck or other neuromodulation apparatus may include a microcontroller or other microprocessor to control neuromodulation. A microcontroller or other microprocessor has myriad uses including, but not limited to: interpreting a received signal and triggering or scheduling a neuromodulation protocol; acquiring and processing a brain recording and/or other physiological measurement; controlling user interface and indicator components; running system checks for device function and safety; confirming that the impedance between one or more pairs of electrodes configured for TES falls below a threshold value chosen from the group of: less than about 250 kΩ, less than about 100 kΩ, less than about 50 kΩ, less than about 25 kΩ, less than about 10 kΩ, less than about 5 kΩ, or less than about 1 kΩ (for a TES puck); and confirming that there is a sufficiently low acoustic impedance between the head and an ultrasound transducer of a neuromodulation system configured for transcranial ultrasound.

A neuromodulation puck or other neuromodulation apparatus may incorporate global positioning system (OPS) hardware or other system so that it can be remotely located. This aspect of the apparatus is optionally configured to disable the neuromodulation system remotely and to locate a lost system from a remote (e.g. web or app) interface. The 'Find my iPhone' app distributed by Apple is an example of a system that remotely locates hardware.

A neuromodulation puck or other neuromodulation system has one or more sensors to determine the position of the system on the head. Sensors incorporated into a neuromodulation puck estimate its absolute position on the head or its relative position to another puck. In this example, one or more sensors are chosen from the group consisting of: gyroscope, accelerometer, barometer, or another sensor used to determine relative distance, orientation, position, or altitude. Data from the one or more sensors is acquired, processed to derive position information, and used to form an estimate of puck position.

In an alternative example for estimating the position on the head of a neuromodulation puck or other neuromodulation system, a user directs a smartphone, tablet, or camera running a customized app at the system on their head, then machine vision algorithms process the picture or video of the system on the user's head to estimate its position. Beneficial examples of the machine vision algorithm incorporate models of the human face and head to define key landmarks on the user, then estimate the neuromodulation system position from its known visual signature. A simple auditory, visual, or other cue can be provided from the app to inform the user whether the system is in an appropriate location for an intended target and cognitive effect. If a neuromodulation system needs to be moved, the app can advise how it should be moved.

In another example, a closed-loop neuromodulation system uses cloud computing to determine parameters of neuromodulation. For example, data from a brain recording or other physiological measurement of a user is transmitted via the Internet to a remote server which processes the recorded data by one or more algorithms, determines an appropriate set of neuromodulation parameters to achieve a desired change in brain function, then transmits a control signal back to the neuromodulation system.

### EXAMPLES

In an exemplary embodiment, students in a yoga class each wear a TES neuromodulation system configured to induce a state of clam. As they enter the class, each student provides their neuromodulation puck hardware identifier to the instructor (or the hardware identifier is identified automatically by proximity or other means) and indicates their preference for having a state of calm induced during the first or second half of the class. The instructor uses his laptop and specialized software to connect wirelessly to each student's neuromodulation puck according to the hardware address provided by each student, and triggers neuromodulation to begin at the specified portion of the class for each student. By using the unique hardware identifiers for each student, the instructor can specify the timing of an induced state of calm on an individual basis. Optionally, users can provide an access code to the teacher that is only temporarily functional, so that it cannot be reused at a later time when the user no longer wishes to grant third party access.

A subscriber identity module (SIM) card is another example of portable and exchangeable hardware identification configurable for use with cellular broadband chipsets. SIM cards enable direct communication with a neuromodulation system through cellular data networks. An advantageous feature of SIM cards is that they can be removed and placed in a different device. Exchanging SIM cards would be advantageous for users traveling to a region that uses a different mobile communication standard. SIM cards can also be configured to be exchanged between a user's different neuromodulation systems. A user who has a transcranial ultrasound neuromodulation puck and a TES neuromodulation puck can use a single SIM card (and thus single neuromodulation 'address') when using one puck or the other.

Bluetooth pairing is a further example used for controlling hardware selectively. From a smartphone or other Bluetooth-enabled device, a customized app or other software is opened. A user indicates her request to pair a neuromodulation puck with the Bluetooth device, then is prompted to press a button or other user interface component on the neuromodulation puck, to confirm which puck is to be paired (a useful feature if more than one neuromodulation puck is within range of the Bluetooth transmitter of the user's smartphone). To confirm successful pairing, the neuromodulation puck can be configured to generate a sound (e.g. chime) from a speaker or flash an LED visual indicator.

Contextual stimulation enables closed-loop examples of the invention. In one example of contextual stimulation, a user's stress levels are monitored by physiological sensors contained as part of a neuromodulation puck, other neuromodulation system, or other hardware system. For instance, stress can be estimated by monitoring heart beats and calculating heart rate variability and/or by measuring galvanic skin resistance. When a user's stress level exceeds a threshold (e.g. as defined by the user at an earlier time or by a therapist or medical professional), a neuromodulation system placed on appropriate head locations and otherwise configured to calm the user is automatically triggered. When the user's stress level drops below a threshold level, neuromodulation stops. Alternatively, a calming neuromodulation session proceeds for a fixed period of time. In another example of a closed-loop system, eye tracking is used to estimate a period of reduced attention from a user, then a neuromodulation system is triggered to focus the subject on a task at hand.

An example of social neuromodulation occurs between two individuals who have smartphones configured for NFC communication and wish to both experience a form of neuromodulation at the same time (e.g. both are at the spa and want to relax together). Each of the users has a smartphone that had been previously linked by a unique (and secure) ID to their particular neuromodulation puck for Bluetooth communication. The users open a customized 'app' designed for social neuromodulation. The app displays a status of the user's neuromodulation puck. Useful messages include: (1) No connection with neuromodulation puck; (2) Low battery on neuromodulation puck; (3) Poor electrical contact by neuromodulation puck to head. (Or, for ultrasound neuromodulation, poor acoustic contact with head.); (4) Warning: do not use transcranial neuromodulation while driving or operating other heavy machinery. Click 'I understand' to continue; (5) Neuromodulation puck is connected. Ready for stimulation. To begin neuromodulation, touch your phone to another phone running the neuromodulation app. Both users need to have the 'Neuromodulation puck is connected' message displayed. When the pair of users touch their phones to each other (or bring them sufficiently close for the NFC sensors to function), the app on both phones displays a 'commencing neuromodulation' message and provides a brief auditory cue 'chime' as feedback.

The methods and apparatuses described herein may be adapted for enhancing musical, political, sporting, and other events experienced in groups or alone by delivering transcranial electrical stimulation (hereinafter 'TES') to one or more individuals experiencing the event.

In general, stimulation by TES may include applying electrical waveforms selected from a list including but not limited to: constant direct current stimulation, pulsed monophasic or biphasic direct current stimulation, alternating current stimulation, cranial electrical stimulation, transcranial random noise stimulation, and other forms of TES. In advantageous examples, stimulation intensity for transcranial constant direct current stimulation is optimally selected to be greater than about 3 mA. In other advantageous examples, stimulation intensity for transcranial pulsed direct current stimulation or alternating current stimulation is optimally selected to be greater than about 5 mA.

For example, members of an audience at a concert, club with a DJ, other musical experience, sporting event, political rally, religious service, or other group experience may use a transcranial electrical stimulation system during the event that induces neuromodulation and enhances the audience members' experience of the event. The venue may be an intimate and small with a small audience (e.g. less than 50 people) or large at an outdoor music festival or stadium (e.g. with an audience exceeding 10,000 people). In at least some instances wherein multiple members of an audience all receive TES, their shared experience of the event is enhanced.

For example, individuals using a neuromodulator (e.g., a TES neuromodulation system) may bring a TES applicator as described herein to the venue and stimulation is triggered to audience members based on proximity, geographic location, or request by the user, e.g. by entering a code into a TES control app running on their smartphone, or by scanning a QR code specific to the event on their smartphone, so that an app can trigger an appropriate waveform at times selected by one or more of the performers or staff of the performance. Wireless communication directly to a TES system is one way to communicate with a TES system so that stimulation can be triggered with timing and waveform selected by one or more of the performers or staff of the performance. In some examples, the system is configured so that a performer (e.g. musician, DJ, dancer, etc.) triggers neuromodulation directly (e.g. with a foot pedal; a button on an electronic instrument such as a synthesizer; or a remote control). In other examples, the system is configured so that a supporting staff member controls neuromodulation of members of the audience, similar to the way that a mixer at a sound board or a person controlling a light show can control delivery of sensory stimuli to the audience. A performer may also wear a neuromodulation apparatus and receive neuromodulation during a performance. The performer's neuromodulation may be concurrent with the audience's neuromodulation or may alternatively occur at a different time. The performer may have their neuromodulation apparatus configured so that the form of neuromodulation they receive is the same as the audience or, alternatively, the form of neuromodulation received by the audience is different than that received by the performer.

Neuromodulation can be similarly configured so that leaders of other forms of group experiences such as sporting events, political rallies, motivational speeches, or religious events control neuromodulation received by members of the audience.

In an alternative example, an individual who wishes to share in the experience of a musical performance or other group experience listens to a recording in their home by themselves or in a small group while wearing a neuromodulation system that is activated at appropriate times relative to the audio so that the user can experience a version of the event without being there, much as listening to a recording of a live concert permits an enjoyable proxy to being at the event.

In another example, attendees at a club with a DJ performing a set of electronic dance music or other music receive a wearable neuromodulation apparatus (e.g., TES neuromodulation apparatus) upon entry to the club, adhere TES electrodes at appropriate locations based on instructions provided by the performer and/or kit, and receive electrical stimulation triggered remotely. An advantageous feature of this system is the capacity to integrate neuromodulation with the musical performance and light show in order to enhance the concert experience.

In another example, a performer or other member of the performance staff defines the timing of neuromodulation and each user controls one or more aspects of the neuromodulation protocol selected from the list including but not limited to: waveform, intensity, cognitive state induced, and length. In an alternative example, the performer or other member of the performance staff defines the timing of neuromodulation as well as an aspect of the waveform (e.g. ramping of the waveform) to control the relative intensity of an induced cognitive effect in a member of the audience.

In yet another example, the timing and other features of neuromodulation controlled by a performer or other member of the performance staff are: (1) controlled in real-time to respond to the energy of the audience; (2) pre-recorded or otherwise pre-determined; or (3) triggered based on a pattern of sound, light, or other stimulus. All participants or attendees may experience the same form, duration, and timing of neuromodulation. For example, one or more of TES form (e.g. change in cognitive state induced), TES duration, and TES timing may differ among members of the audience, for instance in groups, sections, sets, or by demographics of audience members (e.g. males experience one form of TES and females receive a different timing or form of TES).

In some variations, the transcranial neuromodulation apparatus is dermally adhesive and comprised of two assemblies, a master assembly and a slave assembly, each containing a dermally adhesive electrode assembly. Examples include TES systems comprising a first dermally adhesive assembly having at least one electrode and a second dermally adhesive assembly having at least one electrode configured so that the relative position of the two assemblies is only constrained by the length of at least one electrically conductive flexible wire connecting the two assemblies.

A neuromodulation apparatus may include at least one layered electrode assembly comprising components layered relative to the side of the electrode assembly furthest from the dermal surface such that: a first layer provides structural support and comprises at least one electrically conductive path for current to be delivered to electrode layers more proximal to the dermal surface; a second layer spreads current delivered from the at least one electrically conductive path of the first layer to layers more proximal to the dermal surface of the electrode; a third layer is dermally adhesive, electrically conductive, and can be removed from a user's skin manually without leaving significant residue; and a fourth layer is a "peel and stick" backing.

For example a neuromodulation apparatus configured for TES may include an electrode assembly comprising multiple electrode contacts with shapes, sizes, orientations, and compositions to target one or more brain region with transcranial electrical stimulation to achieve a desired change in cognitive state. Some electrode assemblies may be configured to have a notch or opening on the electrode backing that permits improved conformability to a curved portion of the head or other part of the body of a subject.

With respect specifically to TES, certain examples can be used independently or together to reduce pain, irritation, and/or burning in tissue at higher current intensities while achieving desirable changes in a subject's cognitive function, cognitive state, mood, and/or energy levels. Advantageous features may include: (1) pulsed monophasic or biphasic electrical stimulation protocols delivered transdermally; (2) combined alternating current and direct current electrical stimulation protocols delivered transdermally; and (3) combined alternating current and direct current electrical stimulation protocols achieved by concurrently delivering an alternating current electrical stimulation protocol from a first set of electrodes and a direct current electrical stimulation protocol from a second set of electrodes affixed to a subject, delivered transdermally.

In some variations of the transcranial neuromodulation apparatuses configured for TES, alternating current stimulation or pulsed transcranial direct current stimulation (ptDCS; also referred to as monophasic pulsed direct current stimulation) having at least one dominant frequency between 0.5 Hz and 1 MHz is used. tACS or ptDCS protocols may have at least one dominant frequency between about 650 Hz and about 25 kHz. Sensory pathways that transduce perceptions of pain, itching, and irritation are not typically activated with biphasic alternating stimulation at these frequencies. Moreover, pH changes that cause irritation and burning do not occur for zero net current or small net currents (e.g. less than about 1.5 mA).

The simplest form of TES is tDCS. In some variations, the circuitry of the transcranial neuromodulation apparatus can be reduced to a voltage supply (generally 9 V or 12 V); a current regulator to supply constant current as the impedance between an electrode and a subject's head changes slightly (e.g. due to movement, sweating, etc.); and safety circuitry to ensure that spikes of current do not pass into the subject. Several open source tDCS projects have released designs for inexpensive TES systems, including the 'Thinking Cap' from Grindhouse Wetware and the Go Flow. Various commercial and custom systems for triggering a specified stimulus waveform to one or more pairs of TES electrodes have also been described.

Historically, stimulation electrodes used in TES have been relatively large, on the order of about more than 2 cm by 2 cm. The motivation for large electrode pads has been to reduce the tingling, itchy, or painful sensation created at the edge of the electrodes from the generated electric field. For instance, a 3 cm × 4 cm electrode and a 5 cm x 7 cm electrode for stimulating somatosensory cortex has been used. A 'high density' electrode system has been proposed with smaller electrodes and improved coupling of the electrical fields to the scalp in order to reduce discomfort (US patent application number 12/937,950).

tACS may require additional hardware to deliver appropriate waveforms to the electrodes, such as alternating currents at an appropriate frequency. An oscillator, microcontroller, or timing circuit can be used to deliver a desired time-varying stimulation. Alternating current stimulation pulses can comprise square waves, sine waves, sawtooth waves, triangular waves, rectified (unimodal) waves, pulse-width modulated, amplitude-modulated, frequency-modulated, or other pattern of alternating current waveform. System and methods for transcranial alternating current stimulation to induce neuromodulation in a subject is described by Chaieb et al. (Chaieb L, Antal A, Paulus W. "Transcranial alternating current stimulation in the low kHz range increases motor cortex excitability." Restor Neurol Neurosci. 2011;29(3):167-75).

tRNS additionally requires a microcontroller or other processor configured to provide random values with appropriate structure that are then converted to an analog signal and used to gate current at the desired intensity. System and methods for transcranial alternating current stimulation to induce neuromodulation in a subject is described by Saiote et al. (Saiote C, Polanía R, Rosenberger K, Paulus W, Antal A (2013) "High-Frequency TRNS Reduces BOLD Activity during Visuomotor Learning." PLoS ONE 8(3): e5 9669).

Cranial electrotherapy stimulation (CES) delivers pulsed electrical stimulation at a pulse repetition frequency selected to be between about 0.1 Hz and about 200 Hz, preferably within the range of frequencies between about 0.5 Hz and about 100 Hz. A common pulse repetition frequency for CES is 0.5 Hz. Another common pulse repetition frequency for CES is 100 Hz. US patent 6,567,702 to Nekhendzy and Maze describes a transcranial analgesia machine configured to deliver pulsed electrical stimulation having similar frequency as CES.

The waveform of tDCS, tACS, CES, or tRNS delivered to a subject using a transcranial neuromodulation apparatus can be constant or modified in one or more ways selected form the list including, but not limited to, pulsed, ramped, modulated, or interferential. There are many useful waveforms that could be used in tDCS, tACS, CES, or tRNS, and any such waveform may be used with the apparatuses and method described herein.

### CONTROLLERS

As mentioned, any of the transcranial neuromodulation apparatuses described herein may stimulate neural tissue to activate, inhibit, or otherwise modulate the activity of cells in the nervous system and achieve a cognitive effect, change in cognitive state, or other physiological change in a user. These wearable neuromodulation devices may also include a controller (a first party controller) that is paired with and wirelessly controls the transcranial neuromodulation apparatus. Such a first-party controller (e.g., for use by the wearer) may be adapted to also or alternatively operate as a third-party controller that networks with other transcranial neuromodulation apparatuses.

In general a controller (e.g., first-party controller, third-party controller) may be configured to adapt a mobile computing device (such as a smartphone) with software stored on a non-transitory computer readable medium and executable by the mobile computing device that causes the mobile computing device to communicate and control the wearable neuromodulation apparatus.

For example, a wearable neuromodulation apparatus may be a transcranial electrical stimulation (TES) apparatus. Asjust discussed, transcranial electrical stimulation includes forms of electrical stimulation referred to as transcranial direct current stimulation (tDCS), transcranial alternating current stimulation (tACS), targeted electrical stimulation (TES), cranial electrical stimulation (CES), and other forms of transcranial electrical stimulation that affect the activity of cells in the nervous system. In alternative examples of the systems and methods described herein, electrical stimulation is delivered transdermally to a subject to affect a neural target outside the cranium. These secondary examples are non-transcranial and configured to affect the activity of cells in the nervous system.

Mobile computing devices have become constant companions to daily life in many parts of the world, and the proliferation of powerful mobile computing devices will likely continue in the coming years as falling costs open developing markets. Mobile computing devices contain at least one microprocessor, a machine-readable memory, an operating system (e.g. Android, iOS, or Windows Phone), at least one wireless communication module, and a user interface that incorporates a touchscreen and/or mechanical buttons. Mobile computing systems are widely available, including smartphones (Apple iPhone, Android Nexus, Samsung Galaxy, Nokia Lumia and many others), tablet computers (Apple iPad, Samsung Galaxy Note, Microsoft Surface, Amazon Kindle, and many others), smartwatches (Sony SmartWatch, Samsung Galaxy Gear, and many others), and other mobile computing systems (i.e. Apple iPod Touch).

Mobile computing devices are programmable with factory-installed and user-selected application software that are commonly referred to as 'apps'. Thus, as used herein, an 'app' may refer to software stored on a non-transitory computer readable medium on a mobile computing device and executable by the mobile computing device.

The described examples may have significant advantages relative to existing systems including, but not limited to: flexible configurability, portability, computational power, wireless connectivity, and Internet-capability. In certain examples, an App on a mobile computing device causes the mobile computing device to provide one or more user interface elements displayed on a touchscreen and/or using mechanical user interface elements of the mobile computing device (i.e. a button, slider, orjoystick). Mobile computing systems commonly incorporate user interface elements that can be used for communication with and control of a wearable neuromodulation system, including, but not limited to: a touchscreen, mechanical buttons, voice control, sounds, and haptic sensory signals.

Despite the benefits of TES for neuromodulation, existing systems are lacking in at least some instances regarding the efficacy, comfort, and/or convenience of a TES session. Moreover, some other features of a TES system include improved user interface features selected from the list including, but not limited to: user feedback, metadata entry, retrospective (historical) display of one or more TES sessions, display of a prospective (planned) TES session, instructions for placement and orientation of one or more TES electrodes on the user's body (i.e. incorporating augmented reality of an image of a user's head or other body area to assist the user in placing one or more electrodes on a portion of the body the user cannot see such as the neck or forehead), control over waveform characteristics (e.g. intensity, frequency, and/or ramping) with a slider or other user interface component, and coordination of a TES session across multiple users for concurrent neuromodulation.

A networkable transcranial neuromodulation system may comprise a wearable neuromodulation device and a mobile computing device configured with software stored on a non-transitory computer readable medium and executable by the mobile computing device that causes the mobile computing device to achieve one or more actions selected from the list including, but not limited to: establishing a wireless connection to hardware; assisting a user in placing one or more electrodes on the body, for instance by providing instructions on a screen of a mobile computing device; displaying a plurality of neuromodulation protocols available for user selection; receiving user input to select a stimulation protocol; receiving user input to change an intensity, frequency, ramping, or other parameter of a TES session; receiving user input to deliver a transient such as a brief pause in stimulation or phosphene-inducing stimulus; receiving user input to select a desired cognitive effect, change in cognitive state, or change in physiological state during a neuromodulation session; wirelessly transmitting a selected stimulation protocol to a neuromodulation system wearably attached to a user and thereby induce neuromodulation in a neural target in a user; communicating unidirectionally or bidirectionally with a wearable neuromodulation device; transmitting data about a neuromodulation session, including physiological data and/or metadata about the user via the Internet to a remote server; providing search capability for retrospective data about previous neuromodulation sessions by the user or a third party; hosting and/or participating in a group neuromodulation session; displaying data about the status of a connected neuromodulation device (i.e. amount of charge remaining in a battery); receiving user input to repeat a previously experienced neuromodulation protocol; displaying a user interface for the user to provide feedback about the experienced neuromodulation (i.e. to indicate whether an experienced neuromodulation session was a favorite - or by rating the neuromodulation session according to a rating from one to five stars; and sharing information about a neuromodulation session via social media.

In a preferred embodiment, the neuromodulation device connected to the mobile computing device is a transcranial electrical stimulation device. In other embodiments, the neuromodulation device connected to the mobile computing device is a transcutaneous electrical stimulation device with at least one electrode adhered to a user's head, face, or neck and an effect transduced by at least one neural target area. In other embodiments, the wearable neuromodulation device connected to the mobile computing device comprises components for one or more alternative technologies for stimulating neural tissue to activate, inhibit, or modulate the activity of cells in the nervous system selected from the group that includes, but is not limited to: ultrasound neuromodulation, transcranial magnetic stimulation (TMS), deep brain stimulation (DBS), stimulation through one electrode or an array of electrodes implanted on the surface of the brain or dura, and light activation of specially engineered proteins for neuromodulation known as optogenetics.

Hardware and software systems for TES may include: a battery or power supply safely isolated from mains power; control hardware and/or software for triggering a TES event and controlling the waveform, duration, intensity, and other parameters of stimulation of each electrode; and one or more pairs of electrodes with gel, saline, or another material for electrical coupling to the scalp. In alternate embodiments, the hardware and software systems for TES may include additional or fewer components. One of ordinary skill in the art would appreciate hardware and software systems for TES may include a variety of components.

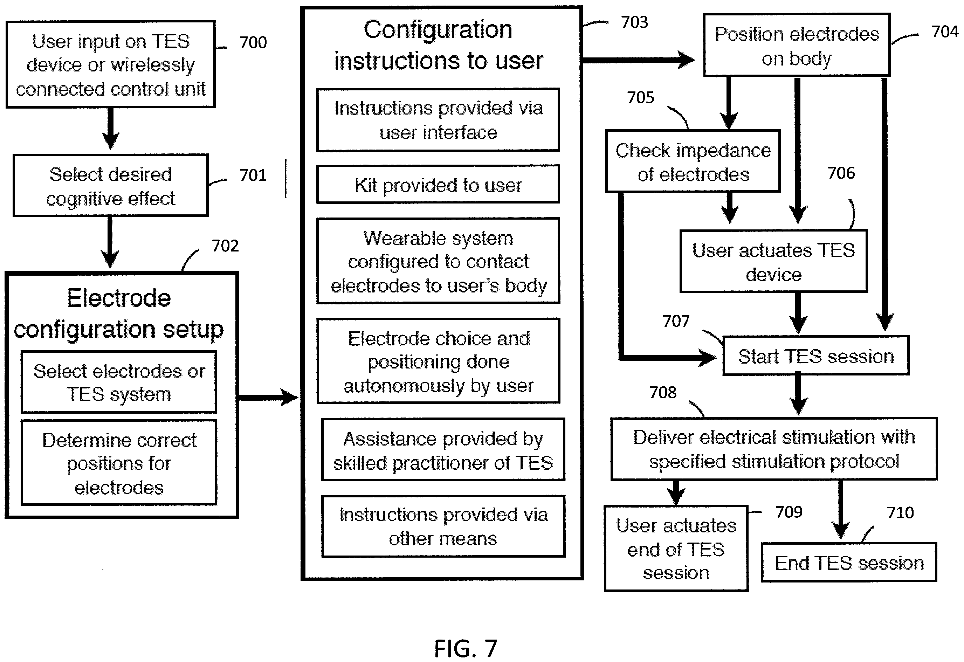

FIG. 7 shows an exemplary workflow for configuring, actuating, and ending a TES session. User input on TES device or wirelessly connected control unit 700 may be used to select desired cognitive effect 701 which determines electrode configuration setup 702 to achieve the desired cognitive effect, including selection of electrodes or a TES system that contains electrodes and determination of correct positions for electrodes. In an example, configuration instructions to user 703 are provided by one or more ways selected from the list including but not limited to: instructions provided via user interface; kit provided to user; wearable system configured to contact TES electrodes to appropriate portions of a user's body; electrode choice and positioning done autonomously by user (e.g. due to previous experience with TES); assistance provided by skilled practitioner of TES; and instructions provided via other means.

Based on these instructions or knowledge, a user or other individual or system positions electrodes on body 704. In some examples, the TES session starts 707 automatically after electrodes are positioned on the body. In other examples, the impedance of the electrodes 705 is checked by a TES system before the TES session starts 707. In some examples, after impedance of the electrodes 705 is checked by a TES system, user actuates TES device 706 before the TES session starts 707. In other examples, after positioning electrodes on the body 704 the user actuates the TES device 706 to start the TES session 707. Once the TES session starts, the next step is to deliver electrical stimulation with specified stimulation protocol 708. In some examples, a user actuates end of TES session 709. In other examples, the TES session ends automatically when the stimulation protocol completes 710.

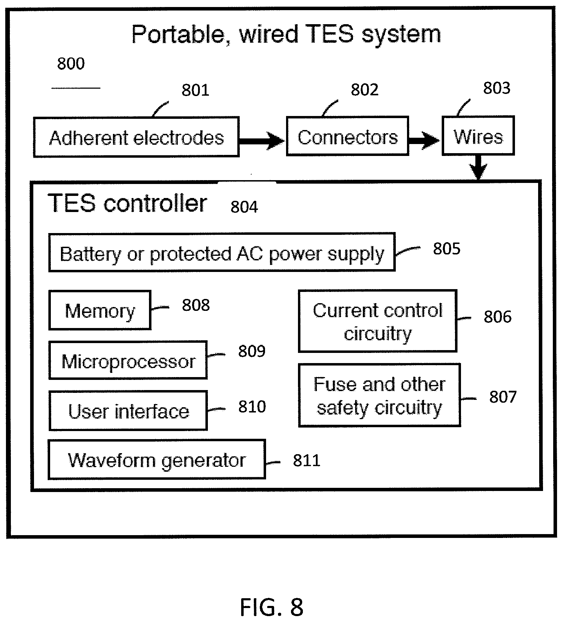

FIG. 8 shows components of portable, wired TES system 800. In this example, adherent electrodes 801 connect to TES controller 804 via connectors 802 and wires 803. TES controller 804 has several components including battery or protected AC power supply 805, fuse and other safety circuitry 807, memory 808, microprocessor 809, user interface 810, current control circuitry 806, and waveform generator 811. The neuroConn DC-stimulator (neuroConn GmbH, Ilmenau, Germany) and Activadose II (Activatek Inc. Salt Lake City, UT) are commercially available portable systems that connect to electrodes by wires that can be used for tDCS. The inTENSity™ product line (Current Solutions LLC, Austin, TX) are commercially available portable systems that connect to electrodes by wires and can be configured for constant and interferential tACS. One skilled in the art will recognize that other commercial or custom systems can be used as a portable, wired TES system to deliver tACS, tDCS, tRNS, or another form of TES.

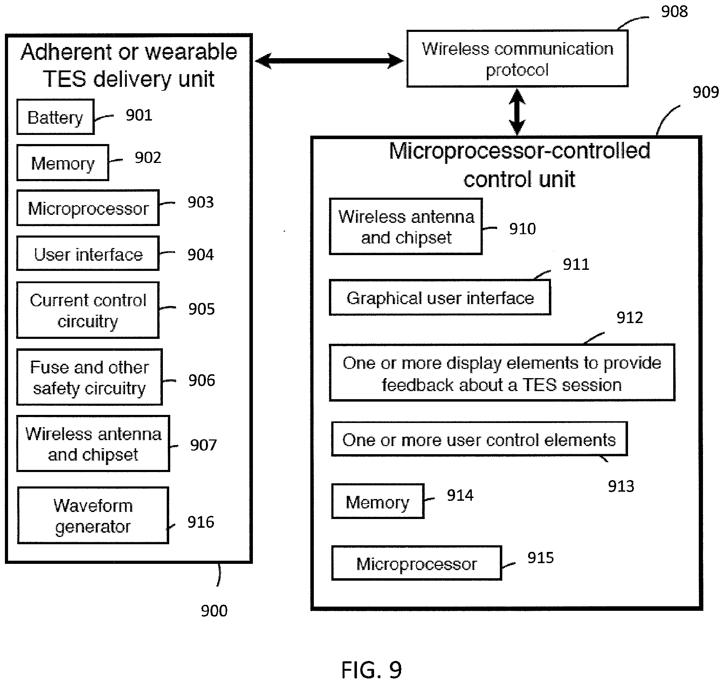

FIG. 9 shows a TES system comprising adherent or wearable TES delivery unit 900 that communicates wirelessly with microprocessor-controlled control unit 909 (e.g. a smartphone running an Android or iOS operating system such as an iPhone or Samsung Galaxy, a tablet such as an iPad, a personal computer including, but not limited to, laptops and desktop computers, or any other suitable computing device). In this exemplar embodiment, adherent or wearable TES delivery unit 900 holds two or more electrodes in dermal contact with a subject with one or more of: an adhesive, a shaped form factor that fits on or is worn on a portion of a user's body (e.g. a headband or around-the-ear 'eyeglass' style form factor). In an exemplar embodiment, adherent or wearable TES delivery 900 comprises components: battery 901, memory 902, microprocessor 903, user interface 904, current control circuitry 905, fuse and other safety circuitry 906, wireless antenna and chipset 907, and waveform generator 916. Microprocessor-controlled control unit 909 includes components: wireless antenna and chipset 910, graphical user interface 911, one or more display elements to provide feedback about a TES session 912, one or more user control elements 913, memory 914, and microprocessor 915. In an alternate embodiment the TES delivery unit 900 may include additional or fewer components. One of ordinary skill in the art would appreciate that a TES delivery unit could be comprised of a variety of components.

Adherent or wearable TES delivery unit 900 may be configured to communicate bidirectionally with wireless communication protocol 908 to microprocessor-controlled system 909. The system can be configured to communicate various forms of data wirelessly, including, but not limited to, trigger signals, control signals, safety alert signals, stimulation timing, stimulation duration, stimulation intensity, other aspects of stimulation protocol, electrode quality, electrode impedance, and battery levels. Communication may be made with devices and controllers using methods known in the art, including but not limited to, RF, WIFI, WiMax, Bluetooth, BLE, UHF, NHF, GSM, CDMA, LAN, WAN, or another wireless protocol. Pulsed infrared light as transmitted for instance by a remote control is an additional wireless form of communication. Near Field Communication (NFC) is another useful technique for communicating with a neuromodulation system or neuromodulation puck. One of ordinary skill in the art would appreciate that there are numerous wireless communication protocols that could be utilized.

Adherent or wearable TES delivery unit 909 may not include user interface 904 and is controlled exclusively through wireless communication protocol 908 to control unit 909. In an alternate embodiment, adherent or wearable TES delivery unit 909 does not include wireless antenna and chipset 907 and is controlled exclusively through user interface 904. One skilled in the art will recognize that alternative TES systems can be designed with multiple configurations while still being capable of delivering electrical stimulation transcranially and transdermally into a subject.

The pattern of currents delivered into tissue of a subject (e.g. transcranially into the brain) may depend on the electrode configuration and stimulation protocol. Electrode configuration may comprise one or more parameters selected from the list including, but not limited to, number of electrodes, positions of electrodes, sizes of electrode, shapes of electrode, composition of electrodes, and anode-cathode pairing of electrodes (i.e. whether a set of electrodes is electrically coupled as an anode or cathode; also whether multiple independent channels of stimulation are present via current sources driving independent anode-cathode sets). A stimulation protocol may define the temporal pattern of current delivered to an anode-cathode set and can incorporate one or more waveform components selected from the list including but not limited to: direct current, alternating current, pulsed current, linear current ramp, nonlinear current ramp, exponential current ramp, modulation of current, and more complex (including repeated, random, pseudo-random, and chaotic patterns).

Current flow at target areas in the brain induces neuromodulation when appropriate electrode configurations and stimulation protocols are delivered. The spatiotemporal of currents in the brain determines whether neuromodulation occurs and, if so, the nature of the change induced.

Described below and in a series of drawings is an example embodiment in which a neuromodulation system comprising a wearable transcranial electrical stimulation (TES) device wirelessly connected to a mobile computing device configured with software stored on a non-transitory computer readable medium and executable by the computing device that causes the computing device to communicate with and control the TES device for delivery neuromodulation to the user wearing the TES device.

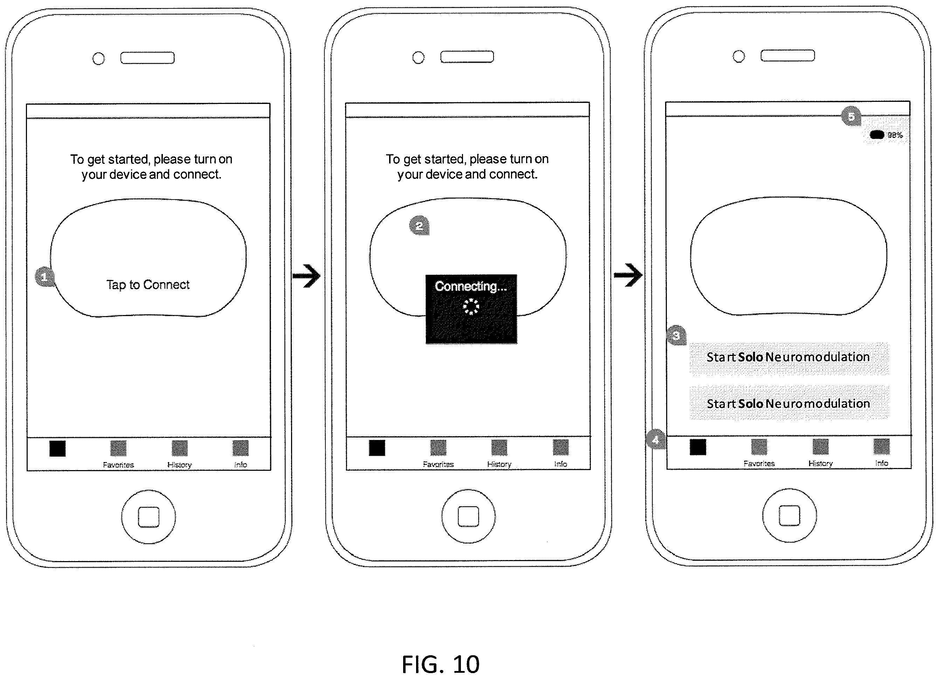

FIG. 10 shows mobile computing device user interface display and functionality for connecting a wearably attached transcranial electrical stimulation device to a mobile computing device caused by software stored on a non-transitory computer readable medium and executable by the mobile computing device. Shown on the left is a default view. User must establish a connection between the neuromodulation puck and the app by tapping 'Tap to Connect'. The center display shows a Spinner that appears while connection is established. The right display shows buttons to start a transcranial electrical stimulation session. If successful, the initial instructions and connect button swap for options to start a solo or group session. The right display also shows TES device battery life when device is connected. Also shown in each of the screens is a global tab navigation that includes a main workflow, Favorites, History, and Info. A field testing version of the app automatically shares session data for diagnostic purposes. A full release version has a Settings tab to opt in or out of sharing.

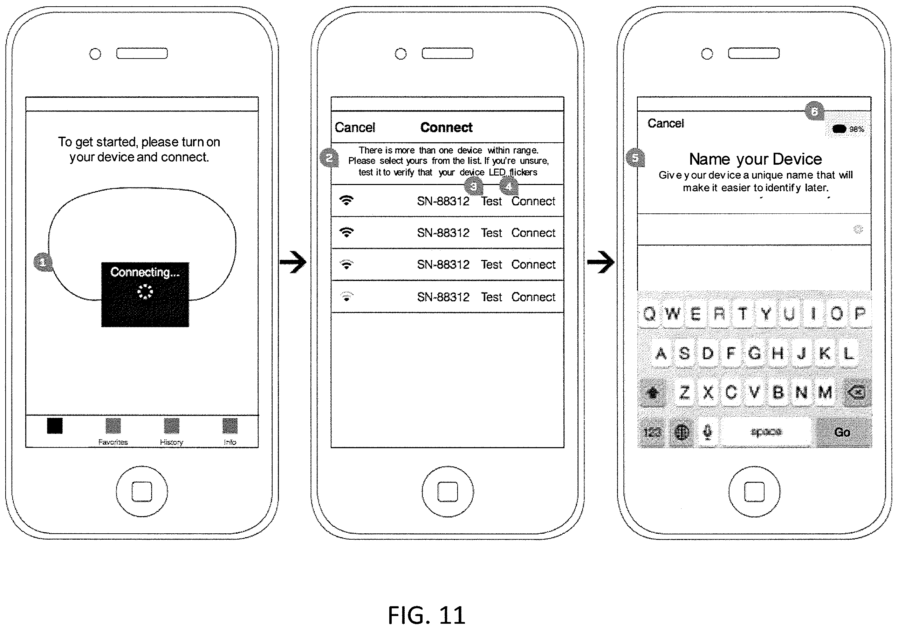

FIG. 11 shows mobile computing device user interface display and functionality for selecting a wearably attached transcranial electrical stimulation device to connect to a mobile computing device caused by software stored on a non-transitory computer readable medium and executable by the mobile computing device. For a TES device connection step the mobile computing device searches for TES devices within range to connect wirelessly and displays a spinner (left screen) while searching. The center screen shows user interface components for selecting a device for connection. A list slides up to reveal options if there is more than one TES device within range. If the mobile computing device has already paired (connected) with a TES device, the software will cause the mobile computing device to attempt to connect with the same product automatically the next time. If the mobile computing device has never previously been paired with that mobile computing device, the software requires users to select from a list of TES devices in range to connect wirelessly (if there is more than one TES device in range). The center display shows a test button that when tapped causes the power LED on the specified TES device to flicker in response as a way to verify which TES device is which. The center display shows a connect button that when tapped causes the mobile computing device to attempt to connect to the specified TES device. The right display shows a user interface including a keyboard for naming the connected TES device for later reference.

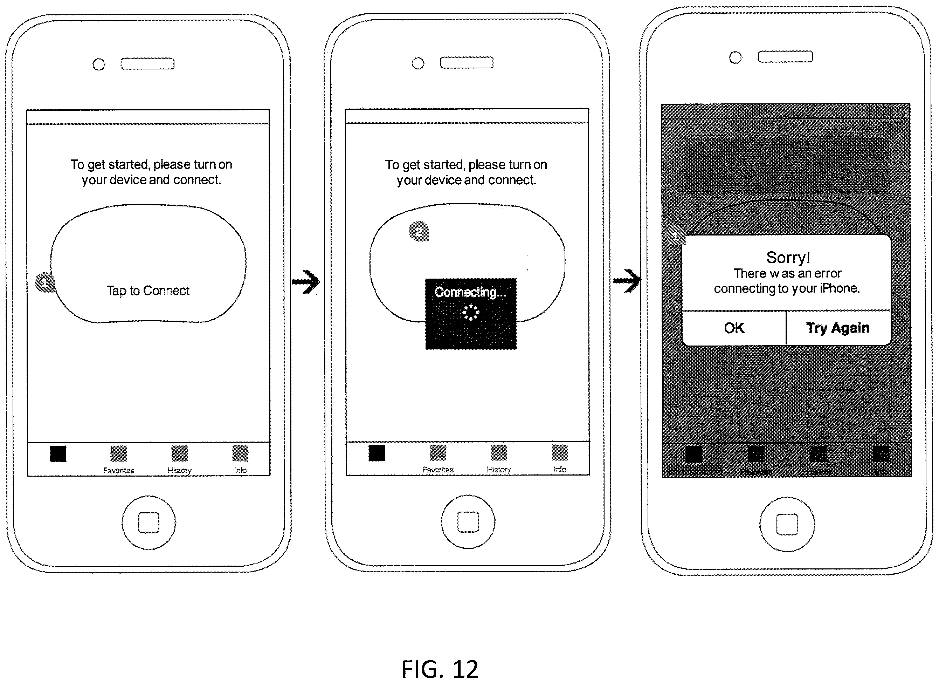

FIG. 12 shows mobile computing device user interface display and functionality for indicating errors have occurred for connecting a wearably attached transcranial electrical stimulation device to a mobile computing device caused by software stored on a non-transitory computer readable medium and executable by the mobile computing device. The series of displays show user interface components indicating that the mobile computing device is attempting to connect to a TES device and a standard modal alert if connection fails, so that a user can provide input to the app to try to connect again or dismiss the connection attempt.

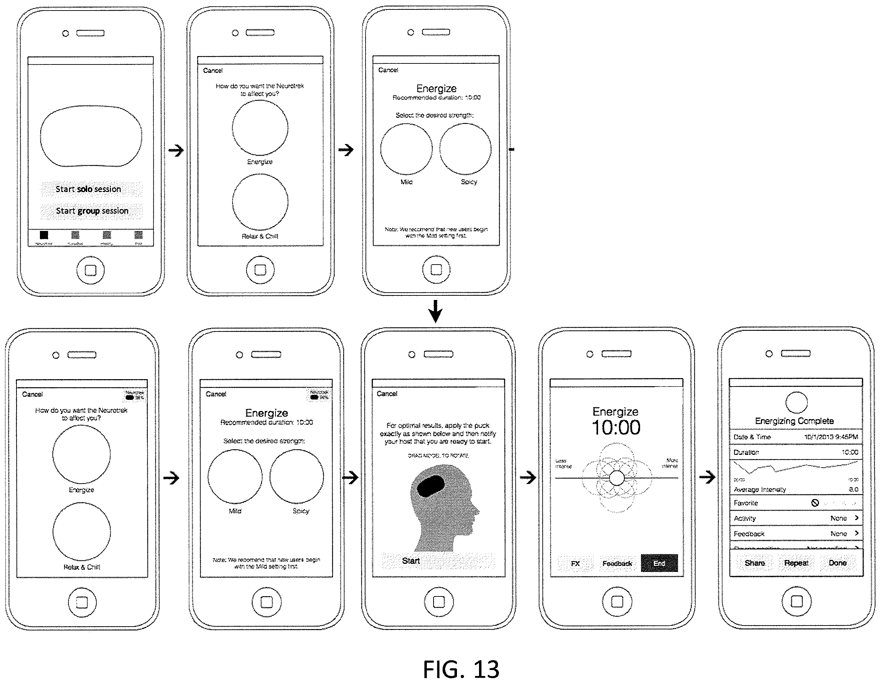

FIG. 13 shows mobile computing device user interface display and functionality to set up, execute, and provide feedback about a solo transcranial electrical stimulation session caused by software stored on a non-transitory computer readable medium and executable by the mobile computing device. On the top row of displays, the display on the left shows a 'start solo Neuromodulation' button tapped by a user who wishes to start a solo TES session. The center display on the top row shows different cognitive states that can be affected by different neuromodulation protocols from the TES device connected to the mobile computing device - and requires the user selects one of the options. The right display on the top row shows user interface buttons for a user to select the duration and intensity of the selected form of neuromodulation to be induced by the TES device. The bottom row of displays shows user interface elements to (from left to right): assist the user in placing electrodes for a desired form of neuromodulation; provide feedback during a TES session to a user; and provide retrospective feedback after a TES session is completed.

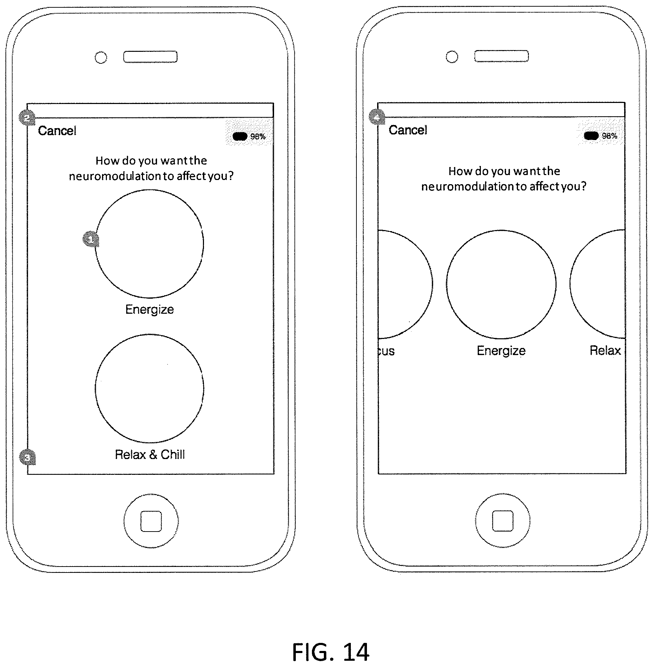

FIG. 14 shows mobile computing device user interface display and functionality for selecting a neuromodulatory effect to be induced by a transcranial electrical stimulation device caused by software stored on a non-transitory computer readable medium and executable by the mobile computing device. Shown are two embodiments of user interface elements permitting a user to select an 'energy' effect or a 'relax & chill' effect.

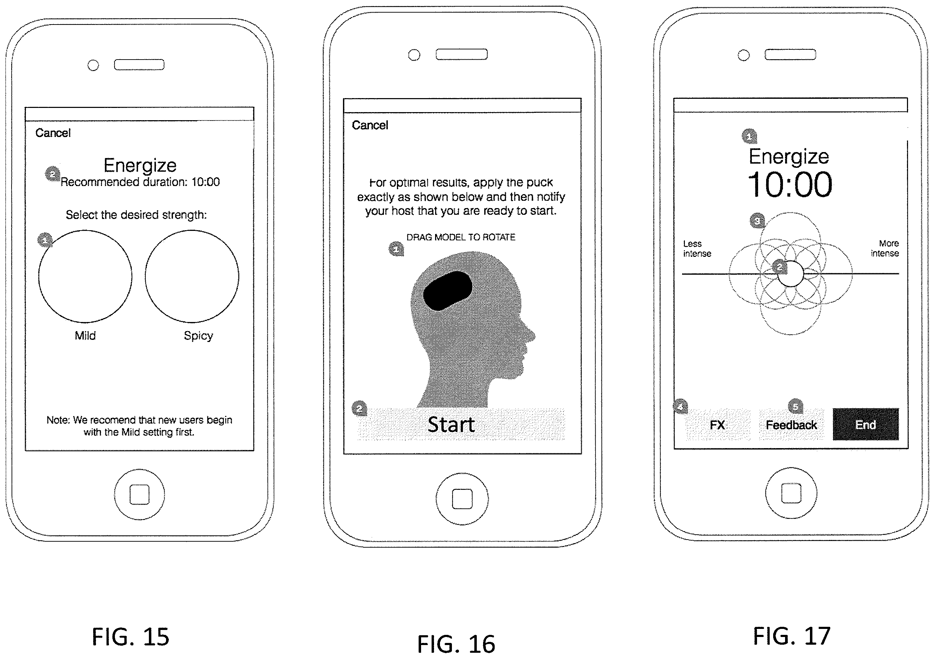

FIG.15 shows mobile computing device user interface display and functionality for selecting the intensity and duration of transcranial electrical stimulation caused by software stored on a non-transitory computer readable medium and executable by the mobile computing.

FIG.16 shows mobile computing device user interface display and functionality for instructing a user on the placement of electrodes of a transcranial electrical stimulation device caused by software stored on a non-transitory computer readable medium and executable by the mobile computing device. In examples, a front-facing camera on a mobile computing device provides the user feedback about the positioning of one or more TES electrodes on a part of the body the user cannot see (e.g. forehead). Augmented reality can be used to show intended positions of the electrodes. Fiduciary markers on the actual electrodes placed by the user can be used with a machine vision protocol to instruct the user how to shift the electrodes to appropriate positions.

FIG.17 shows mobile computing device user interface display and functionality for controlling a transcranial electrical stimulation protocol during a neuromodulation session caused by software stored on a non-transitory computer readable medium and executable by the mobile computing. A slider permits a user to change the intensity of stimulation (and additional sliders or other user interface components can be added to provide control of frequency, ramping, or other parameters).

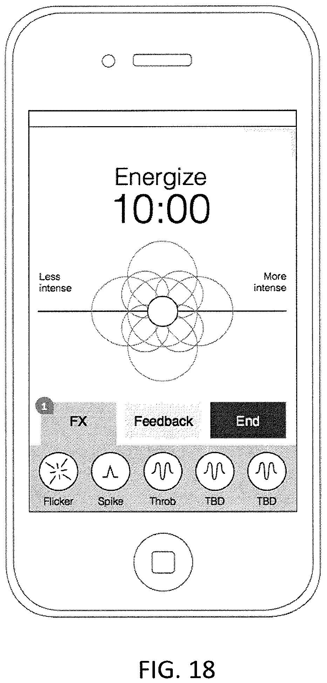

FIG. 18 shows mobile computing device user interface display and functionality for selecting an effect to be delivered to a user by transcranial electrical stimulation device caused by software stored on a non-transitory computer readable medium and executable by the mobile computing device. Three exemplar effects are: throb (transiently modulate the intensity and/or frequency of stimulation), flicker (transmit a phosphene), and spike (transiently increase the intensity of stimulation). Other effects are also possible, including an effect that transiently decreases the intensity of stimulation in order to provide a more extreme subjective experience of the induced neuromodulation.

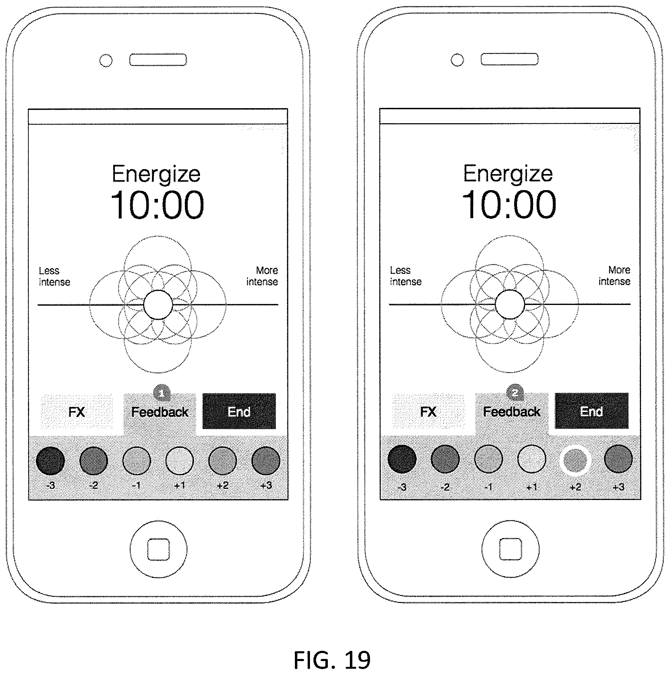

FIG. 19 shows mobile computing device user interface display and functionality for a user to provide feedback during a transcranial electrical stimulation session caused by software stored on a non-transitory computer readable medium and executable by the mobile computing device. Shown are two screens accessed via a tab for providing feedback by the user about the quality of the neuromodulation session for future analysis. In an example, feedback can be provided at any time during a TES session and the timestamp of that stimulation saved by the software (and, optionally, transmitted via the internet to a remote server).

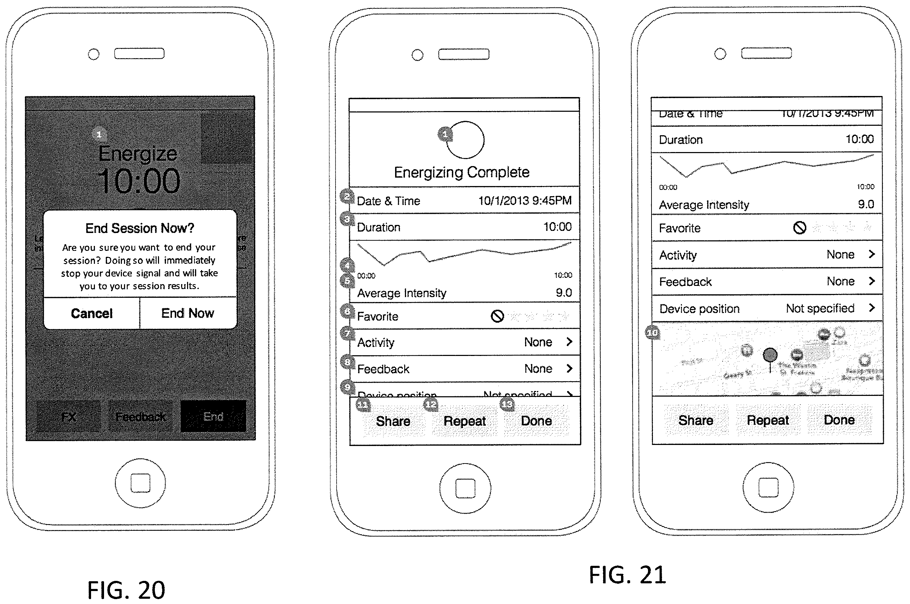

FIG. 20 shows mobile computing device user interface display and functionality for a user to stop a transcranial electrical stimulation session caused by software stored on a non-transitory computer readable medium and executable by the mobile computing device.

FIG. 21 shows mobile computing device user interface display and functionality for providing retrospective data about a transcranial electrical stimulation session caused by software stored on a non-transitory computer readable medium and executable by the mobile computing device.

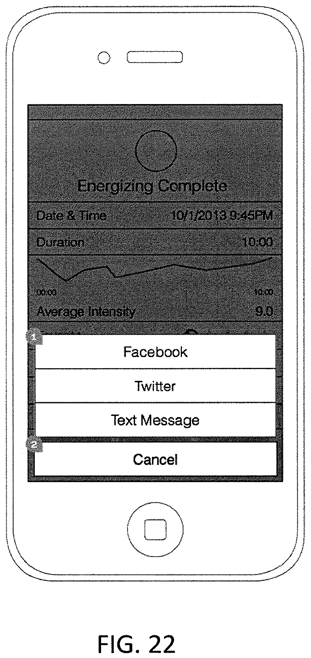

FIG. 22 shows mobile computing device user interface display and functionality for a user to share information about a transcranial electrical stimulation via social media caused by software stored on a non-transitory computer readable medium and executable by the mobile computing device. Exemplar forms of social media by which information about a neuromodulation session can be shared include text message, Facebook, Twitter, and Instagram, although any form of electronic sharing is also applicable. A user selects a network by which to share information and the software automatically creates a message and causes it to be sent to the subject's social media stream based on previous entry of credentials for that social media account by the user.

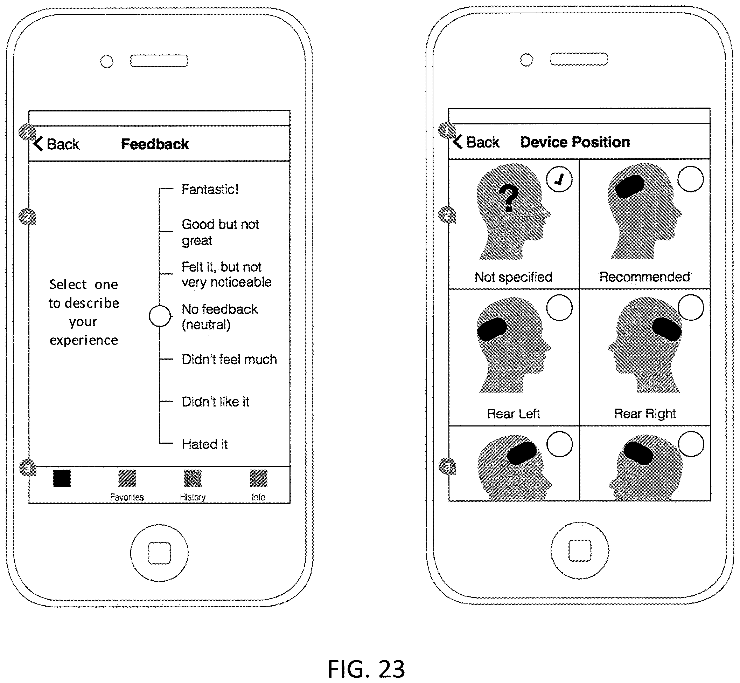

FIG. 23 shows mobile computing device user interface display and functionality for a user to retrospectively provide feedback about the experience of and electrode positions for a transcranial electrical stimulation session caused by software stored on a non-transitory computer readable medium and executable by the mobile computing device.

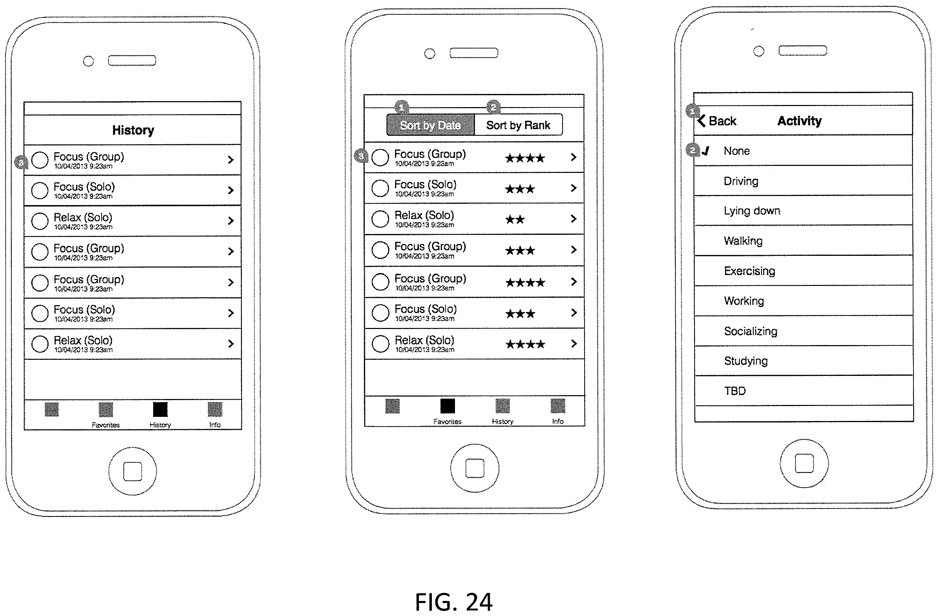

FIG. 24 shows mobile computing device user interface display and functionality showing a historical list of transcranial electrical stimulation sessions caused by software stored on a non-transitory computer readable medium and executable by the mobile computing device. The display on the left shows historical TES sessions sorted by recency. The center display shows historical TES sessions sorted by the feedback score provided by the user. Other data types can also be used for searching, filtering, and sorting historical data, including an entry of metadata concerning concurrent activities by a user as shown in the screen on the right.

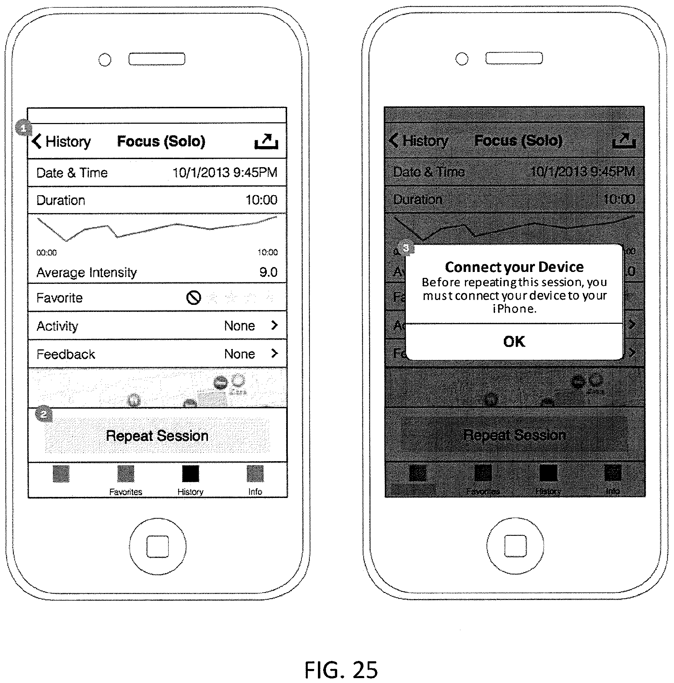

FIG. 25 shows mobile computing device user interface display and functionality for selecting a previously experienced transcranial electrical stimulation session and triggering it to repeat caused by software stored on a non-transitory computer readable medium and executable by the mobile computing device.

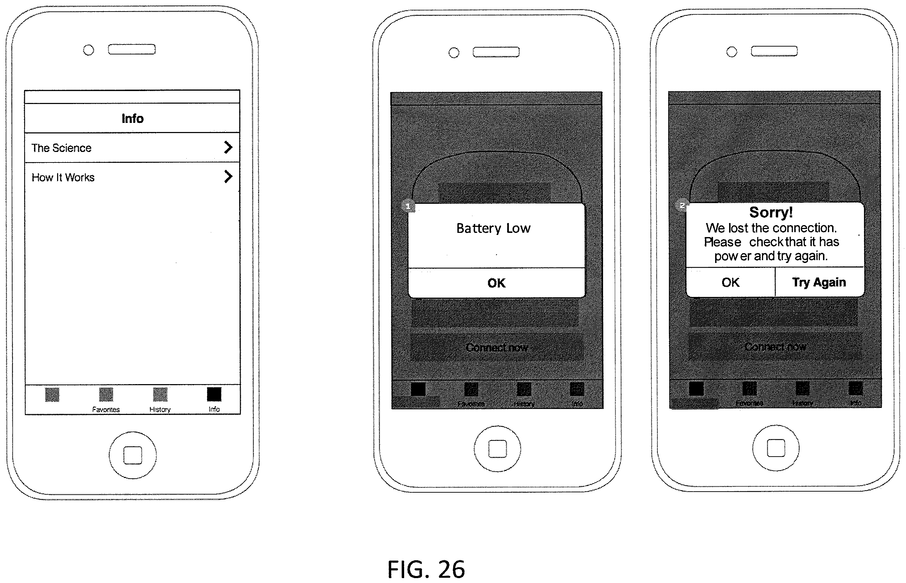

FIG. 26 shows mobile computing device user interface display and functionality for providing descriptive information and error signal notices caused by software stored on a non-transitory computer readable medium and executable by the mobile computing device.

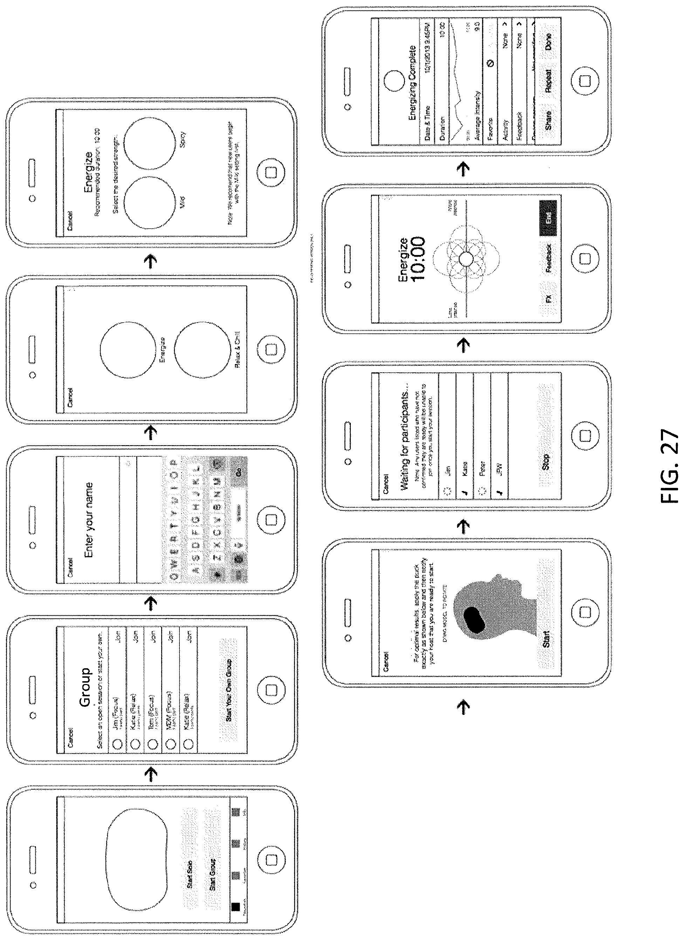

FIG. 27 shows mobile computing device user interface display and functionality to set up (as a host), execute, and provide feedback about a group transcranial electrical stimulation session caused by software stored on a non-transitory computer readable medium and executable by the mobile computing device.

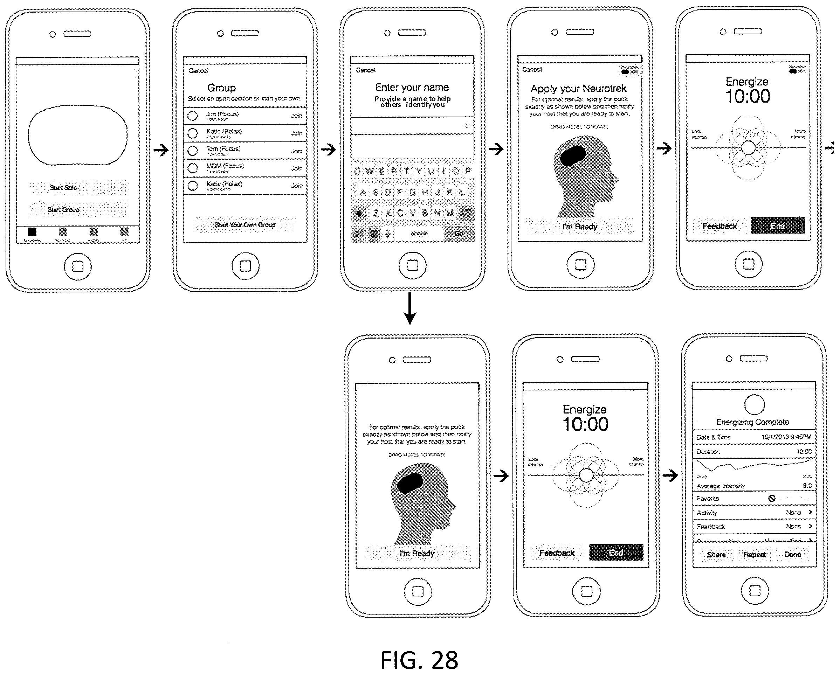

FIG. 28 shows mobile computing device user interface display and functionality to set up (as a participant), execute, and provide feedback about a group transcranial electrical stimulation session caused by software stored on a non-transitory computer readable medium and executable by the mobile computing device.

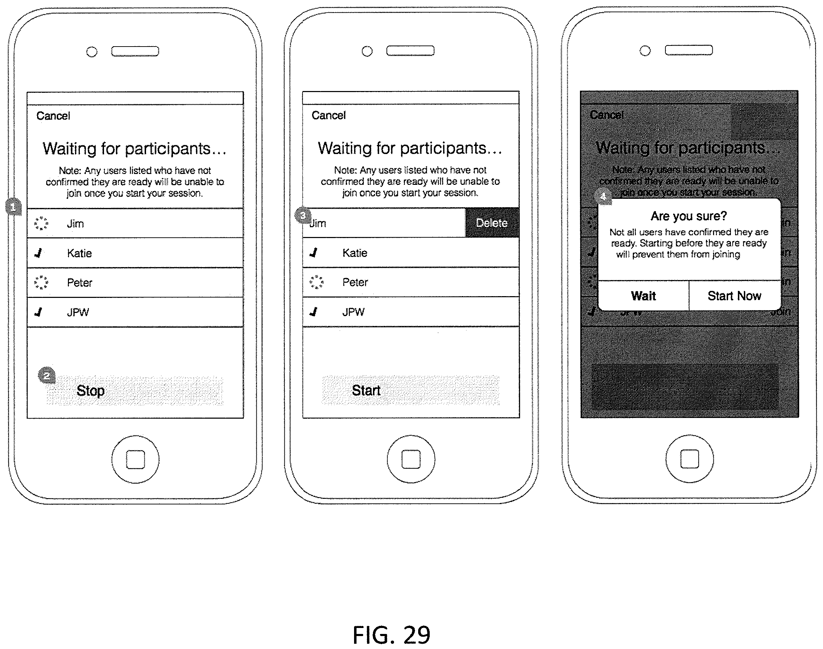

FIG. 29 shows mobile computing device user interface display and functionality to wait for participants to join a group transcranial electrical stimulation session caused by software stored on a non-transitory computer readable medium and executable by the mobile computing device.

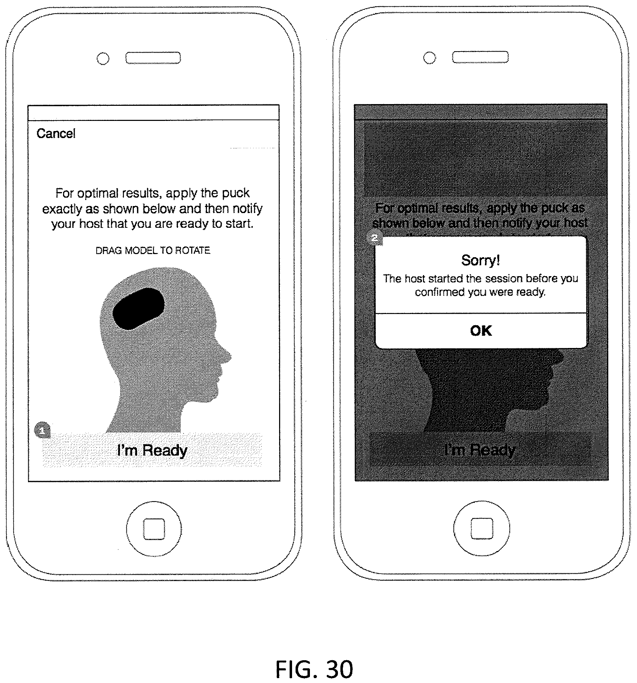

FIG. 30 shows mobile computing device user interface display and functionality for a user in a group transcranial electrical stimulation session to indicate they are ready to join the group session caused by software stored on a non-transitory computer readable medium and executable by the mobile computing device.

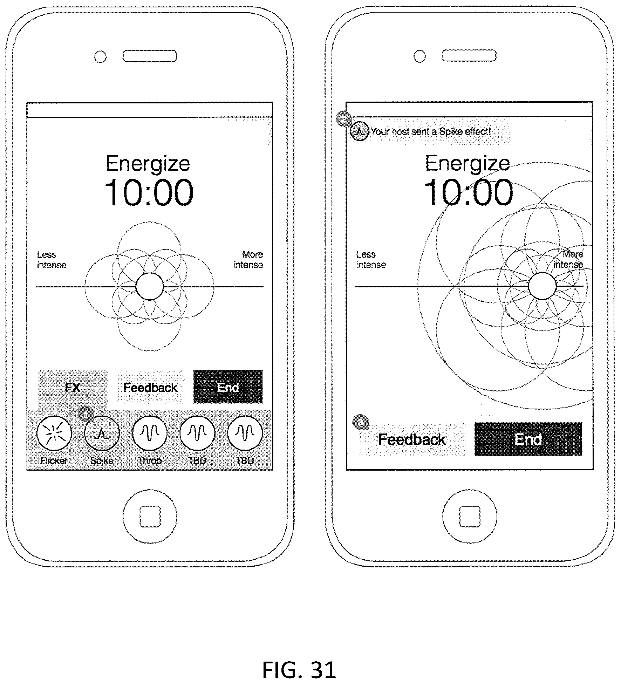

FIG. 31 shows mobile computing device user interface display and functionality for the host of a group transcranial electrical stimulation session to send an effect to the transcranial electrical stimulation device worn by a participant in a group transcranial electrical stimulation session caused by software stored on a non-transitory computer readable medium and executable by the mobile computing device.

As used herein, the term "transcranial neuromodulation system" may refer to a device, assembly, or system for delivering energy transcranially to excite, inhibit, or modulate the activity of a neural circuit. The terms "social neuromodulation session" and "social neuromodulation" may refer to paired, group, and social applications of one or more neuromodulation systems, e.g., where individual neuromodulation is coordinated (e.g., synchronized, duplicated, etc.

The term "mutual TES" may refer to forms of TES wherein transmitted electrical stimulation requires direct (i.e. physical) or indirect electrically conductive contact between two or more individuals in order to close an electrical circuit for TES neuromodulation. The term "self-contained" may refer to a feature of a system or assembly wherein all components of the system or assembly are incorporated in a single housing or enclosure.

The term "self-powered" may refer to a feature of a self-contained system or assembly wherein power is provided by one or more energy sources incorporated in the self-contained system and no external energy source provides power to the self-contained system or assembly. The term "self-adhering" may refer to a feature of a self-contained system or assembly wherein at least one component of the self-contained system or assembly is configured to cause the self-contained system or assembly to adhere to the head in order to successfully deliver a transcranial neuromodulation session and "adhere" is defined as in the Merriam-Webster dictionary as "to hold fast or stick by or as if by gluing, suction, grasping, or fusing". The term "self-coupling" may refer to a feature of a self-contained system or assembly wherein at least one component of the self-contained system or assembly is configured to couple ultrasound energy to the head by forming a low acoustic impedance contact between an ultrasound transducer and the head of the user.

The term "transcranial neuromodulation puck" may refer to an apparatus (e.g., device or system) for transcranial neuromodulation which has one or more properties selected from the group comprising: self-contained, self-powered, and self-adhering. A "transcranial ultrasound neuromodulation puck" may refer to a device for transcranial ultrasound neuromodulation which has one or more properties selected from the group comprising: self-contained, self-powered, self-adhering, and self-coupling. A "transcranial electrical stimulation puck" may refer to a device for transcranial electrical stimulation which has one or more properties selected from the group comprising: self-contained, self-powered, and self-adhering.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements, these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical range recited herein is intended to include all sub-ranges subsumed therein.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. A networkable transcranial neuromodulation system adapted to apply coordinated neuromodulation to a plurality of individuals, the system comprising:
a first transcranial neuromodulator apparatus (900) comprising: a first applicator configured to deliver transcranial neuromodulation to a first individual; a first wireless receiver and transmitter module (907) connected to a first controller (903) configured to apply transcranial neuromodulation from the first applicator and to switch between an autonomous operation mode and a coordinated operation mode;
a second transcranial neuromodulator apparatus (900) comprising: a second applicator configured to deliver transcranial neuromodulation to a second individual; a second wireless receiver and transmitter module (907) connected to a second controller (903) configured to apply transcranial neuromodulation from the second applicator and to switch between an autonomous operation mode and the coordinated operation mode; and
a third-party controller (909) configured to transmit control information to the first and second transcranial neuromodulator apparatuses to coordinate neuromodulation by the first and second transcranial neuromodulator apparatuses when said first and second transcranial neuromodulator apparatuses are in the coordinated operation mode.

2. The system of claim 1, wherein the first transcranial neuromodulator apparatus (900) further comprises a first applicator surface configured to secure the first transcranial neuromodulator apparatus to the first individual's head, wherein the first individual is one from the plurality of individuals; and wherein the second transcranial neuromodulator apparatus (900) further comprises a second applicator surface configured to secure the second transcranial neuromodulator apparatus to the second individual's head, wherein the second individual is one from the plurality of individuals.

3. The system of claim 1, wherein the first and second controllers (903) further comprise a security module configured to determine if a valid security key has been received by the first and second wireless receiver and transmitter modules (907) and to permit switching to the coordinated operation mode only when the valid security key has been received.

4. The system of claim 1, wherein the first transcranial neuromodulator apparatus (900) further comprises a first unique address associated with the first transcranial neuromodulator apparatus, wherein the first controller (903) is configured to apply transcranial neuromodulation based on instructions received during the coordinated operation mode only when the received instructions specify the first unique address associated with the first transcranial neuromodulator apparatus; and wherein, the second transcranial neuromodulator apparatus (900) further comprises a second unique address associated with the second transcranial neuromodulator apparatus, wherein the second controller (903) is configured to apply transcranial neuromodulation based on instructions received during the coordinated operation mode only when the received instructions specify the second unique address associated with the second transcranial neuromodulator apparatus.

5. The system of claim 1, wherein the first and second transcranial neuromodulator apparatuses (900) are configured as transcranial electrical stimulation (TES) apparatuses, and further wherein each of the first and second applicators comprises a pair of electrodes.

6. The system of claim 1, wherein the first and second transcranial neuromodulator apparatuses (900) are configured as combined transcranial electrical stimulation and transcranial ultrasound apparatuses.

7. The system of claim 1, wherein the first and second transcranial neuromodulator apparatuses (900) are configured to transmit a ready status indicator when said first and second transcranial neuromodulator apparatuses are ready to receive instructions from the third-party controller (909).

8. The system of claim 1, wherein each of the first and second transcranial neuromodulator apparatuses (900) further comprises a sensor configured to detect a physiological parameter from an individual.

9. The system of claim 1, wherein each of the first and second transcranial neuromodulator apparatuses (900) further comprises a sensor configured to detect brain activity.

## Patentansprüche

1. Ein netzwerkfähiges transkraniales Neuromodulationssystem, das angepasst ist, um eine koordinierte Neuromodulation auf eine Vielzahl von Individuen anzuwenden, wobei das System aufweist:
eine erste transkraniale Neuromodulatorvorrichtung (900) aufweisend: einen ersten Applikator, der konfiguriert ist, um transkraniale Neuromodulation an ein erstes Individuum bereitzustellen; ein erstes drahtloses Empfänger- und Sendermodul (907), das mit einer ersten Steuerung (903) verbunden sind, die konfiguriert ist, um transkraniale Neuromodulation von dem ersten Applikator anzuwenden und um zwischen einem autonomen Betriebsmodus und einem koordinierten Betriebsmodus umzuschalten;
eine zweite transkraniale Neuromodulatorvorrichtung (900), aufweisend: einen zweiten Applikator, der konfiguriert ist, um transkraniale Neuromodulation an ein zweites Individuum bereitzustellen; ein zweites drahtloses Empfänger- und Sendermodul (907), das mit einer zweiten Steuerung (903) verbunden ist, die konfiguriert ist, um eine transkraniale Neuromodulation von dem zweiten Applikator anzuwenden und um zwischen einem autonomen Betriebsmodus und einem koordinierten Betriebsmodus umzuschalten; und
eine Fremd-Steuerung (909), die konfiguriert ist, um Steuerinformationen an die erste und die zweite transkraniale Neuromodulatorvorrichtungen zu senden, um die Neuromodulation durch die erste und die zweite transkraniale Neuromodulatorvorrichtungen zu koordinieren, wenn die erste und zweite transkraniale Neuromodulatorvorrichtungen in dem koordinierten Betriebsmodus sind.

2. Das System nach Anspruch 1, wobei die erste transkraniale Neuromodulatorvorrichtung (900) weiterhin eine erste Applikatoroberfläche aufweist, die konfiguriert ist, um die erste transkraniale Neuromodulatorvorrichtung an den Kopf des ersten Individuums zu befestigen, wobei das erste Individuum eines aus der Vielzahl von Individuen ist; und wobei die zweite transkraniale Neuromodulatorvorrichtung (900) weiterhin eine zweite Applikatoroberfläche aufweist, die konfiguriert ist, um die zweite transkraniale Neuromodulatorvorrichtung an den Kopf des zweiten Individuums zu befestigen, wobei das zweite Individuum eines aus der Vielzahl von Individuen ist.

3. Das System nach Anspruch 1, wobei die erste und die zweite Steuerungen (903) weiterhin ein Sicherheitsmodul aufweisen, welches konfiguriert ist, um zu bestimmen ob ein gültiger Sicherheitsschlüssel von den ersten und zweiten drahtlosen Empfänger- und Sendermodulen (907) empfangen wurde und um das Umschalten in den koordinierten Betriebsmodus nur zuzulassen, wenn der gültige Sicherheitsschlüssel empfangen wurde.

4. Das System nach Anspruch 1, wobei die erste transkraniale Neuromodulatorvorrichtung (900) weiterhin eine erste eindeutige Adresse aufweist, welche der ersten transkranialen Neuromodulatorvorrichtung zugeordnet ist, wobei die erste Steuerung (903) konfiguriert ist, um die transkraniale Neuromodulation basierend auf während des koordinierten Betriebsmodus empfangenen Anweisungen anzuwenden, nur wenn die empfangenen Anweisungen die erste eindeutige Adresse spezifizieren, die dem ersten transkranialen Neuromodulatorvorrichtung zugeordnet ist; und wobei die zweite transkraniale Neuromodulatorvorrichtung (900) weiterhin eine zweite eindeutige Adresse aufweist, die der zweiten transkranialen Neuromodulatorvorrichtung zugeordnet ist, wobei die zweite Steuerung (903) konfiguriert ist, um die transkraniale Neuromodulation basierend auf während des koordinierten Betriebs empfangenen Anweisungen anzuwenden, nur wenn die empfangenen Anweisungen die zweite eindeutige Adresse spezifizieren, die der zweiten transkranialen Neuromodulatorvorrichtung zugeordnet ist.

5. Das System nach Anspruch 1, wobei die erste und die zweite transkraniale Neuromodulatorvorrichtungen (900) als transkraniale elektrische Stimulationsvorrichtungen (TES-Vorrichtungen) konfiguriert sind, und weiterhin jede der ersten und zweiten Applikatoren ein Paar von Elektroden aufweist.

6. Das System nach Anspruch 1, wobei die erste und zweite transkranialen Neuromodulatorvorrichtungen (900) als kombinierte transkraniale elektrische Stimulations- und transkraniale Ultraschallvorrichtungen konfiguriert sind.

7. Das System nach Anspruch 1, wobei die erste und zweite transkraniale Neuromodulationsvorrichtungen (900) konfiguriert sind, um eine Bereitschaftsstatus-Anzeige zu senden, wenn die erste und zweite transkraniale Neuromodulationsvorrichtungen bereit sind, Anweisungen von einer Fremd-Steuerung (909) zu empfangen.

8. Das System nach Anspruch 1, wobei jede von den ersten und zweiten transkranialen Neuromodulatorvorrichtungen (900) weiterhin einen Sensor aufweisen, der konfiguriert ist, um physiologische Parameter von einem Individuum zu erfassen.

9. Das System nach Anspruch 1, wobei jede von den ersten und der zweiten transkranialen Neuromodulatorvorrichtungen (900) weiterhin einen Sensor aufweisen, der zum Erfassen von Gehirnaktivität konfiguriert ist.

## Revendications

1. Système de neuromodulation transcrânienne pouvant être mis en réseau adapté pour appliquer une neuromodulation coordonnée à une pluralité d'individus, le système comprenant :
un premier appareil neuromodulateur transcrânien (900) comprenant : un premier applicateur configuré pour délivrer une neuromodulation transcrânienne à un premier individu ; un premier module de récepteur et d'émetteur sans fil (907) connecté à un premier dispositif de commande (903) configuré pour appliquer une neuromodulation transcrânienne à partir du premier applicateur et pour effectuer une commutation entre un mode de fonctionnement autonome et un mode de fonctionnement coordonné ;
un second appareil neuromodulateur transcrânien (900) comprenant : un second applicateur configuré pour délivrer une neuromodulation transcrânienne à un second individu ; un second module de récepteur et d'émetteur sans fil (907) connecté à un second dispositif de commande (903) configuré pour appliquer une neuromodulation transcrânienne à partir du second applicateur et pour effectuer une commutation entre un mode de fonctionnement autonome et le mode de fonctionnement coordonné ; et
un dispositif de commande tiers (909) configuré pour transmettre des informations de commande aux premier et second appareils neuromodulateurs transcrâniens pour coordonner une neuromodulation par les premier et second appareils neuromodulateurs transcrâniens lorsque lesdits premier et second appareils neuromodulateurs transcrâniens sont dans le mode de fonctionnement coordonné.

2. Système selon la revendication 1, dans lequel le premier appareil neuromodulateur transcrânien (900) comprend en outre une première surface d'applicateur configurée pour fixer le premier appareil neuromodulateur transcrânien à la tête du premier individu, dans lequel le premier individu est l'un de la pluralité d'individus ; et dans lequel le second appareil neuromodulateur transcrânien (900) comprend en outre une seconde surface d'applicateur configurée pour fixer le second appareil neuromodulateur transcrânien à la tête du second individu, dans lequel le second individu est l'un de la pluralité d'individus.

3. Système selon la revendication 1, dans lequel les premier et second dispositifs de commande (903) comprennent en outre un module de sécurité configuré pour déterminer si une clé de sécurité valide a été reçue par les premier et second modules de récepteur et d'émetteur sans fil (907) et pour permettre une commutation au mode de fonctionnement coordonné uniquement lorsque la clé de sécurité valide a été reçue.

4. Système selon la revendication 1, dans lequel le premier appareil neuromodulateur transcrânien (900) comprend en outre une première adresse unique associée au premier appareil neuromodulateur transcrânien, dans lequel le premier dispositif de commande (903) est configuré pour appliquer une neuromodulation transcrânienne sur la base d'instructions reçues pendant le mode de fonctionnement coordonné uniquement lorsque les instructions reçues spécifient la première adresse unique associée au premier appareil neuromodulateur transcrânien ; et dans lequel, le second appareil neuromodulateur transcrânien (900) comprend en outre une seconde adresse unique associée au second appareil neuromodulateur transcrânien, dans lequel le second dispositif de commande (903) est configuré pour appliquer une neuromodulation transcrânienne sur la base d'instructions reçues pendant le mode de fonctionnement coordonné uniquement lorsque les instructions reçues spécifient la seconde adresse unique associée au second appareil neuromodulateur transcrânien.

5. Système selon la revendication 1, dans lequel les premier et second appareils neuromodulateurs transcrâniens (900) sont configurés sous la forme d'appareils de stimulation électrique transcrânienne (TES), et en outre dans lequel chacun des premier et second applicateurs comprend une paire d'électrodes.

6. Système selon la revendication 1, dans lequel les premier et second appareils neuromodulateurs transcrâniens (900) sont configurés sous la forme d'appareils combinés de stimulation électrique transcrânienne et d'ultrasons transcrâniens.

7. Système selon la revendication 1, dans lequel les premier et second appareils neuromodulateurs transcrâniens (900) sont configurés pour transmettre un indicateur d'état prêt lorsque lesdits premier et second appareils neuromodulateurs transcrâniens sont prêts à recevoir des instructions depuis le dispositif de commande tiers (909).

8. Système selon la revendication 1, dans lequel chacun des premier et second appareils neuromodulateurs transcrâniens (900) comprend en outre un capteur configuré pour détecter un paramètre physiologique sur un individu.

9. Système selon la revendication 1, dans lequel chacun des premier et second appareils neuromodulateurs transcrâniens (900) comprend en outre un capteur configuré pour détecter une activité cérébrale.
